# EUROPEAN PATENT SPECIFICATION

(11) **EP 3 033 430 B1**
(45) Date of publication and mention of the grant of the patent: **26.09.2018**
(21) Application number: 14744073.9
(22) Date of filing: 24.07.2014
(51) Int. Cl.: C12Q 1/6886

(54) **COMBINATIONS OF MOLECULAR MARKERS IN PROSTATE CANCER PROVIDING A DIAGNOSTIC TOOL WITH IMPROVED SENSITIVITY/SPECIFICITY**
KOMBINATIONEN VON MOLEKULARMARKERN IN PROSTATAKREBS ZUR BEREITSTELLUNG EINES DIAGNOSEWERKZEUGS MIT VERBESSERTER EMPFINDLICHKEIT/SPEZIFITÄT
COMBINAISONS DE MARQUEURS MOLÉCULAIRES DU CANCER DE LA PROSTATE, PERMETTANT D'OBTENIR UN OUTIL DE DIAGNOSTIC AYANT UNE SENSIBILITÉ/SPÉCIFICITÉ AMÉLIORÉES

(30) Priority: 13.08.2013 WO PCT/EP2013/066889
(43) Date of publication of application: 22.06.2016
(73) Proprietor: MDxHealth Research B.V., 6525 GA Nijmegen (NL)
(72) Inventor: SMIT, Franciscus Petrus, NL-6546 RN Nijmegen (NL); HESSELS, Daphne, NL-6546 RN Nijmegen (NL); SCHALKEN, Jacobus A., NL-6524 LH Nijmegen (NL)
(74) Representative: van Kooij, Adriaan
(86) International application number: PCT/EP2014/065899
(87) International publication number: WO 2015/022164

(56) References cited:
- WO-A1-2012/152800
- WO-A1-2012/152811
- WO-A1-2013/064636
- MILLER GARY J ET AL: "Aberrant HOXC expression accompanies the malignant phenotype in human prostate", CANCER RESEARCH, AMERICAN ASSOCIATION FOR CANCER RESEARCH, US, vol. 63, no. 18, 15 September 2003 (2003-09-15), pages 5879-5888, XP002476391, ISSN: 0008-5472

## Description

The present invention relates to methods for *in vitro* establishing, or diagnosing, high grade or low grade prostate cancer in a sample, preferably from a readily obtainable sample such as an urine, a prostatic fluid or ejaculate sample or a processed, or derived sample thereof, originating from human individual suspected of suffering from prostate cancer using expression level analysis of a combination of four molecular markers for prostate cancer. The present invention further relates to the use in expression level analysis of these combined markers for *in vitro* establishing high grade or low grade prostate cancer and to a kit of parts providing expression analysis of combinations of the present molecular markers for establishing high grade or low grade prostate cancer.

In the Western male population, prostate cancer has become a major public health problem. In many developed countries it is not only the most commonly diagnosed malignancy, but it is the second leading cause of cancer related deaths in males as well. Because the incidence of prostate cancer increases with age, the number of newly diagnosed cases continues to increase as the life expectancy of the general population increases. In the United States, approximately 218,000 men, and in Europe approximately 382,000 men are newly diagnosed with prostate cancer every year.

Epidemiology studies show that prostate cancer is an indolent disease and that more men die with prostate cancer than from it. However, a significant fraction of the tumors behave aggressively and as a result approximately 32,000 American men and approximately 89,000 European men die from this disease on a yearly basis.

The high mortality rate is a consequence of the fact that there are no curative therapeutic options for metastatic prostate cancer. Androgen ablation is the treatment of choice in men with metastatic disease. Initially, 70 to 80% of the patients with advanced disease show response to therapy, but with time the majority of the tumors will become androgen independent. As a result most patients will develop progressive disease.

Since there are no effective therapeutic options for advanced prostate cancer, early detection of this tumor is pivotal and can increase the curative success rate. Although the routine use of serum prostate-specific antigen (PSA) testing has undoubtedly increased prostate cancer detection, one of the main drawbacks of the serum PSA (sPSA) test is the low specificity. Also conditions such as benign prostatic hyperplasia (BPH) and prostatitis can lead to an elevated sPSA level. This results in high negative biopsy rates of 70-80% in the so-called 'grey area' of PSA levels 4.0-10.0 ng/ml.

Moreover, PSA-based screening has led to the diagnosis of clinically insignificant prostate tumors, i.e. in the absence of screening, these tumors would not have been diagnosed within the patient's lifetime, which results in over-treatment.

Therefore, (non-invasive) molecular tests, that can accurately identify those men who have early stage, clinically localized prostate cancer and who would gain prolonged survival and quality of life from early radical intervention, are urgently needed. The prime challenge for molecular diagnostics is the identification of clinically significant prostate cancer, *i.e.* a Gleason Score of >=7 and/or percentage biopsy positive cores >=33% and/or clinical stage >=T2 (Epstein criteria). Furthermore, markers predicting and monitoring the response to treatment are urgently needed. Molecular biomarkers identified in tissues can serve as target for new body fluid based molecular tests. A suitable biomarker preferably fulfils the following criteria:
1) it must be reproducible (intra- en inter-institutional); and
2) it must have an impact on clinical management.
Further, for diagnostic purposes, it is important that the biomarkers are tested in terms of tissue-specificity and discrimination potential between prostate cancer, normal prostate and BPH. Furthermore, it can be expected that (multiple) biomarker-based assays enhance the sensitivity for cancer detection.

Considering the above, there is an urgent need for molecular prognostic biomarkers capable of predicting the biological behaviour of prostate cancer and outcome.

For the identification of new candidate markers for prostate cancer, it is necessary to study expression patterns in malignant as well as non-malignant prostate tissues, preferably in relation to other medical data.

Recent developments in the field of molecular techniques have provided new tools that enabled the assessment of both genomic alterations and proteomic alterations in samples in a comprehensive and rapid manner. These tools have led to the discovery of many new promising biomarkers for prostate cancer. These biomarkers may be instrumental in the development of new tests that have a high specificity in the diagnosis and prognosis of prostate cancer.

For the molecular diagnosis of prostate cancer, genes that are highly up-regulated in prostate cancer compared to low or normal expression in normal prostate tissue are of special interest. Such genes could enable the detection of one tumor cell in a large background of normal cells, and could thus be applied as a diagnostic marker in prostate cancer detection.

In the search for PCa specific biomarkers, two promising candidates have been identified: Prostate CAncer gene 3 (*PCA3*) and TMPRSS2-ERG gene fusions. These biomarkers can be measured using a non-invasive urine test. The *PCA3* gene is highly over-expressed in prostate tumors, and has diagnostic value to predict biopsy outcome, but its prognostic value is limited. The Progensa® PCA3 test is a FDA-approved molecular diagnostic test that is available to urologists.

Gene fusions in which ETS family members are mostly fused to androgen-regulated genes, particularly *TMPRSS2,* are PCa-specific molecular events. TMPRSS2-ERG gene fusions are present in approximately 50% of PCa patients. The prognostic value of this gene fusion is still unclear. Consequently, the urgent need for more accurate *prognostic* biomarkers for PCa persists.

In the art, there is a continuing need for assays providing establishment, or diagnosis, of all prostate cancers with maximal sensitivity, specificity, positive predictive value (PPV) and negative predictive value (NPV). All prostate cancers include both low-grade (Gleason Score >7) and high-grade (Gleason Score >=7) prostate cancers and will be further referred to as PrCa_total.

Furthermore, there is a continuing need for assays for the prediction, or prognosis, of clinical significant prostate cancer, i.e. a Gleason Score of >=7 and/or a percentage biopsy positive cores >=33% and/or a clinical stage >=T2 (Epstein criteria) with maximal sensitivity, specificity, positive predictive value (PPV) and negative predictive value (NPV). These clinically significant prostate cancers will be further referred to as Sign-PrCa.

The present invention disclosed a two, three or four gene based model for improved diagnosis of PrCa_total and/or Sign-PrCa for meeting the above indicated needs of the art.

Sensitivity relates to the assay's ability to identify positive results. In the present context, sensitivity indicates the proportion of individuals suffering from prostate cancer testing positive for PrCa_total or Sign-PrCa.

Specificity relates to the ability of the test to identify negative results. In the present context, specificity is defined as the proportion of individuals not suffering from prostate cancer (based on negative prostate biopsies) testing negative for PrCa_total or Sign-PrCa.

Positive predictive value (PPV) relates to the ability of a test to identify the proportion of positive test results that are true positives. In the present context, PPV is defined as the proportion of individuals with prostate cancer (PrCa_total or Sign-PrCa) testing correctly positive among all positive test results.

Negative predictive value (NPV) relates to the ability of a test to identify the proportion of negative test results that are true negatives. In the present context, NPV is defined as the proportion of individuals without prostate cancer (i.e. negative prostate biopsies) testing correctly negative among all negative test results.

WO 2013/064636 discloses methods for in vitro establishing, or diagnosing, high grade or low grade prostate cancer in a sample, preferably from a readily obtainable sample such as an urine, a prostatic fluid or ejaculate sample or a processed, or derived sample thereof, originating from human individual suspected of suffering from prostate cancer using expression level analysis of a combination of two, or three molecular markers for prostate cancer selected from DLX1, HOXC6 and HOXD10.

WO 2012/152811 discloses methods for *in vitro* diagnosing prostate cancer in a human individual comprising: 1) determining the expression of one or more genes chosen from the group consisting of ACSM1, ALDH3B2, CGREF1, COMP, C19orf48, DLX1, GLYATL1, MS4A8B, NKAIN1, PPFIA2, PTPRT, TDRD1 and/or UGT2B15; and 2) establishing up regulation of expression of said one or more genes as compared to expression of the respective one or more genes in a sample from an individual without prostate cancer thereby providing said diagnosis of prostate cancer.

WO 2012/152800 discloses methods for establishing the presence, or absence, of prostate cancer in a human individual comprising: a) determining the expression of HOXC4 in a sample originating from said human individual; b) establishing up, or down, regulation of expression of HOXC4 as compared to expression of HOXC4 in a sample originating from said human individual not comprising prostate tumour cells or prostate tumour tissue, or from an individual not suffering from prostate cancer; and c) establishing the presence, or absence, of prostate cancer based on the established up-or down regulation of HOXC4.

It is an object of the present invention, amongst other objects, to provide an assay for establishing, or diagnosing, PrCa_total or Sign-PrCa in a sample of a human individual suspected to suffer from prostate cancer thereby aiding in the development of an effective clinical strategy to treat prostate cancer.

The above object, amongst other objects, is met by the present invention as outlined in the appended claims providing an assay and means for performing the assay allowing detecting, amongst others, PrCa_total or Sign-PrCa with improved sensitivity, specificity, PPV and NPV.

Specifically, the above object, amongst other objects, is met, according to a first aspect of the present invention, by methods for *in vitro* establishing prostate cancer (PrCa) in a sample originating from a human individual suspected of or suffering from prostate cancer comprising:
a) determining expression levels of DLX1 and HOXC6;
b) establishing up-regulation of the expression levels of DLX1 and HOXC6 as compared to expression levels of DLX1 and HOXC6 in a sample originating from an individual not suffering from prostate cancer or as compared to a reference value indicative of a non-disease expression level; and
c) establishing the presence or absence of prostate cancer (PrCa) based on the up-regulation of the expression levels of DLX1 and HOXC6 in said sample.
wherein step (a) further comprises determining the expression levels of HOXC4 and TDRD1; wherein step (b) further comprises establishing up-regulation of the expression levels of HOXC4 and TDRD1 as compared to expression levels of HOXC4 and TDRD1 in a sample originating from an individual not suffering from prostate cancer or as compared to a reference value indicative of a non-disease expression level; and wherein step (c) further comprises establishing the presence or absence of prostate cancer (PrCa) based on the up-regulation of the expression levels of DLX1, HOXC6, HOXC4 and TDRD1 in said sample.

In the present description, reference is made to human genes, such as DLX1 and HOXC6, suitable as biomarkers for prostate cancer by referring to their arbitrarily assigned names. Although the skilled person is readily capable to identify, and use, the present genes as biomarkers based on these names, the appended sequence listing provides both the cDNA sequence and protein sequences of these genes in the public database. Based on the data provided in the tables and figures, the skilled person, without undue experimentation and using standard molecular biology means, will be capable of determining the expression levels of the indicated biomarkers in a sample thereby providing the present methods.

In the present description, prostate biopsies are considered to be the gold standard for PrCa diagnosis. Negative biopsies indicate normal prostate conditions and will be further referred to as no-PrCa.

In the present description, expression level analysis comprises establishing an increased expression of least biomarkers HOXC4, HOXC6, DLX1 and TDRD1 as compared to expression of these genes in a similar, equivalent, or corresponding sample originating from a human individual not suffering from prostate cancer (no-PrCa). In other words, an increased expression level of a gene or biomarker according to the present invention is a measure of gene expression relative to a non-disease standard.

Establishing an increased expression of biomarkers HOXC4, HOXC6, DLX1 and TDRD1 as compared to expression of these genes under non-prostate cancer conditions (no-PrCa), allows establishing, or diagnosing prostate cancer (PrCa_total) or significant prostate cancer (Sign-PrCa), thereby providing prognosis and/or prediction of disease survival and an aid to design a clinical treatment protocol.

HOXC4 and HOXC6 are family members of the homeobox superfamily of genes and the HOX subfamily contain members that are transcription factors involved in controlling and coordinating complex functions during development via spatial and temporal expression patterns. In humans, there are 39 classical HOX genes organized into the clusters A, B, C and D.

HOXC4, is one of several homeobox HOXC genes located in a cluster on chromosome 12. Three genes, HOXC4, HOXC5 and HOXC6, share a 5' non-coding exon. Transcripts may include the shared exon spliced to the gene-specific exons, or they may include only the gene-specific exons. Two alternatively spliced variants that encode the same protein have been described for HOXC4. Transcript variant one represents the longer transcript and includes the shared exon. Transcript variant two includes only gene-specific exons and differs in the 5'UTR compared to variant 1. Within the context of the present invention, HOXC4 expression level determination refers to the expression levels of variants one and two.

Also for HOXC6, alternatively spliced transcript variants encoding different isoforms have been identified. Transcript variant two represents the longer transcript and includes the shared exon. It contains a distinct 5'UTR and lacks an in-frame portion of 5' coding region compared to variant one. The resulting isoform two has a shorter N-terminus when compared to isoform one. Transcript variant one includes only gene-specific exons and encodes the longer isoform. Within the context of the present invention, HOXC6 expression level determination refers to the expression levels of variants 1 and 2.

TDRD1 is a tudor related gene essential for male germ-cell differentiation. Tudor domains are found in many eukaryotic organisms and have been implicated in protein-protein interactions in which methylated protein substrates bind to these domains. TDRD1 plays a central role during spermatogenesis by participating in the repression transposable elements and prevent their mobilization, which is essential for the germline integrity.

DLX1 belongs to the family of homeodomain transcription factors which are related to the Drosophila distal-less (Dll) gene. The family has been related to a number of developmental features and appears to be well preserved across species. Dlx genes are implicated in tangential migration of interneurons from the subpallium to the pallium during vertebrate brain development. It has been suggested that Dlx promotes the migration of interneurons by repressing a set of proteins that are normally expressed in terminally differentiated neurons and act to promote the outgrowth of dendrites and axons.

With respect to DLX1 expression, at least two transcript variants are known. Transcript variant 1 is longer than transcript variant 2 and contains an internal exon in the coding region that results in a frame shift and premature stop codon. Within the context of the present invention, DLX1 expression level determination refers to determination of the expression levels of both transcripts.

According to a preferred embodiment of this first aspect of the present invention, determining expression levels comprises determining mRNA expression levels. In other words, determining expression levels comprises determining transcription levels.

According to another preferred embodiment of this first aspect of the present invention, determining expression levels comprises determining protein levels. In other words, determining expression levels comprises determining translation levels.

According to other particularly preferred embodiments of this first aspect of the present invention, establishing prostate cancer (PrCa) comprises establishing high grade (Gleason score >= 7) or low grade (Gleason score <7) prostate cancer and/or establishing prostate cancer (PrCa) comprises establishing a percentage of positive cores of >=33% and/or establishing prostate cancer (PrCa) comprises establishing a clinical stage of >= T2 according to the Epstein criteria.

According to the present invention, the present methods are preferably performed using a sample selected from the group consisting of urine, urine derived, prostatic fluid, prostatic fluid derived, ejaculate and ejaculate derived, an urine, or an urine derived, sample. These samples are the most readily obtainable samples of human bodily derivable samples.

Within the context of the present description, an urine, prostatic fluid or ejaculate derived sample is a sample originating from these bodily fluids, i.e. sample of these fluids further processed, for example, by sedimentation, extraction, precipitation, dilution etc.

According to a second aspect, the present invention relates to the use of a combination of DLX1, HOXC6, HOXC4 and TDRD1 expression markers for *in vitro* establishing prostate cancer (PrCa) or PrCa_total, preferably Sign_PrCa.

According to a preferred embodiment of this second aspect of the present invention, establishing prostate cancer comprises establishing prostate cancer in a sample selected from the group consisting of urine, urine derived, prostatic fluid, prostatic fluid derived, ejaculate and ejaculate derived.

According to a third aspect, the present invention relates to Use of a kit of parts in a method according to any one of the claims 1 to 7 for *in vitro* establishing prostate cancer (PrCa) in a sample originating from a human individual suspected of or suffering from prostate cancer, the kit comprises:
- expression level analysis means for determining the expression levels of HOXC6 and DLX1;
- instructions for use;
the kit further comprising expression level analysis means for determining the expression levels of HOXC4 and TDRD1.

In the present kits of parts, the expression level analysis means allow detection and quantification of the gene mRNA expression levels of the indicated gene combinations of HOXC4, HOXC6, DLX1 and TDRD1 using any non-invasive molecular biology technique suitable for the purposes of the invention, such as, for example, expression micro-arrays, quantitative real-time PCR, conventional PCR, NASBA, etc.

Quantitative real-time PCR (qRT-PCR) is preferably used according to the present invention to detect and quantify the present diagnostic and/or prognostic genes. This technique is accurate and it allows quantifying the specific mRNA of the genes of interest.

In a particular advantageous embodiment, the invention provides methods and means for determining whether a sample is to be classified as no prostate cancer no-PrCa (prostate biopsies negative for prostate cancer), PrCa_total (all prostate cancers; low- and high grade) or Sign-PrCa (significant prostate cancer: Gleason Score of >=7 and/or a percentage biopsy positive cores >=33% and/or a clinical stage >=T2).

Binary logistic regression analysis can be used to define the best gene panel for the most reliable classification of the patient samples in no prostate cancer, prostate cancer total (diagnosis) or clinical significant prostate cancer (prognosis). The goal of this binary logistic regression analysis is to find the best set of genes so that cases that belong to a particular category of prostate cancer will have a very high calculated probability that they will be allocated to that category.

Using this analysis, combinations of biomarkers HOXC6, DLX1, HOXC4 and TDRD1 can be identified for the diagnosis of prostate cancer (i.e. PrCa_total) and prognosis (i.e. Sign-PrCa) of prostate cancer.

The present invention will be further elucidated in the following detailed examples. In the examples reference is made to figures, wherein:

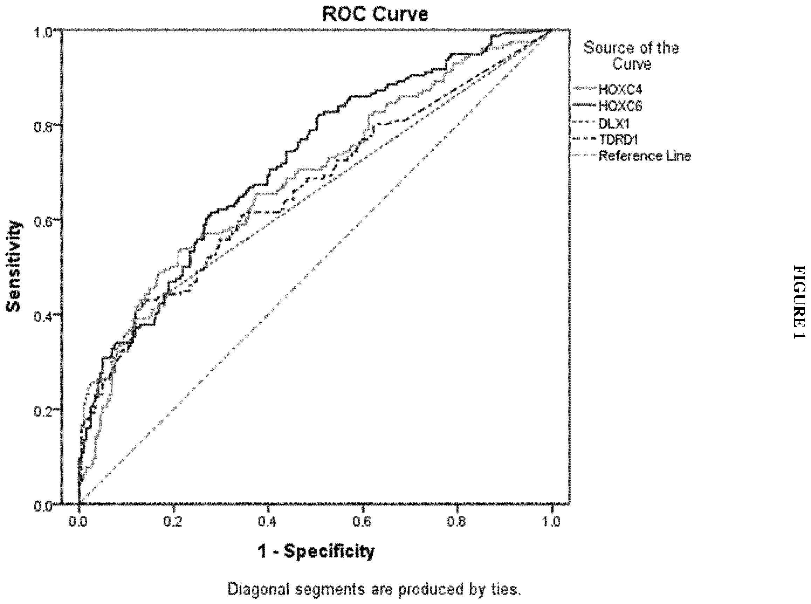

- **Figure 1**: shows the Receiver Operating Characteristic curve to visualize the diagnostic potential of the individual biomarkers HOXC4, HOXC6, DLX1 and TDRD1 to discriminate PrCa_total from no PrCa in the absence of an arbitrary cut-off value.

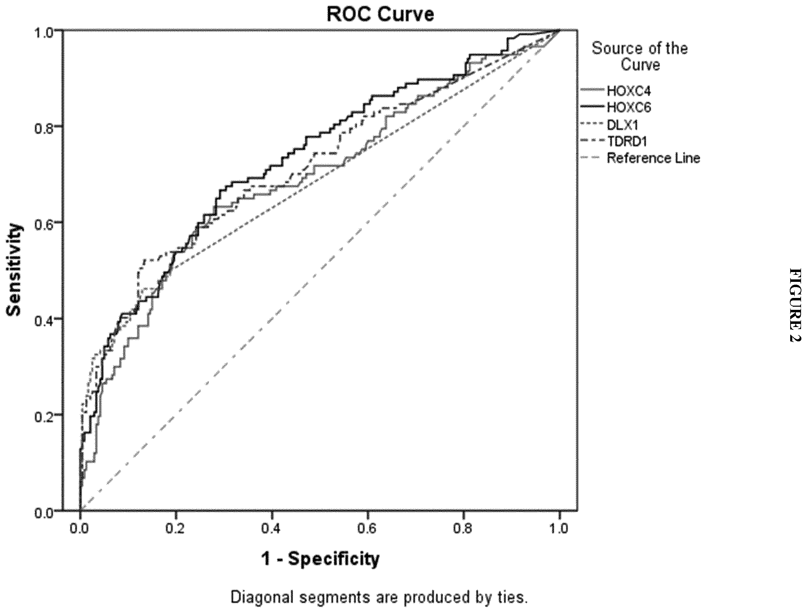

- **Figure 2**: shows the Receiver Operating Characteristic curve to visualize the prognostic potential of the individual biomarkers HOXC4, HOXC6, DLX1 and TDRD1 to discriminate Sign-PrCa from the Rest (i.e. negative biopsy and insignificant PCa) in the absence of an arbitrary cut-off value. Insignificant PrCa is defined as tumors with GS<7, biopsy positive cores <33% and < cT2 stage.

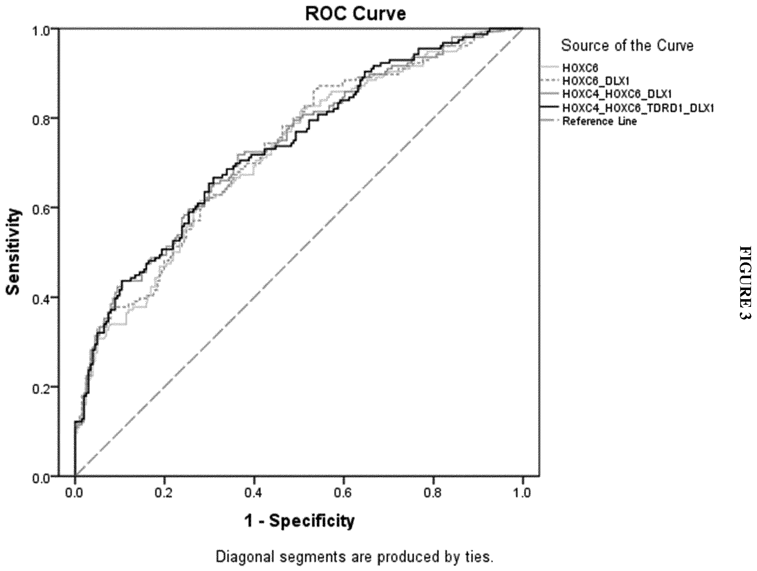

- **Figure 3**: shows the Receiver Operating Characteristic curve to visualize the diagnostic potential of HOXC6, the combination of HOXC6 with DLX1, the combination of HOXC6 with DLX1 and HOXC4 and the combination of HOXC6 with DLX1 and HOXC4 and TDRD1 to discriminate PrCa_total from no PrCa in the absence of an arbitrary cut-off value.

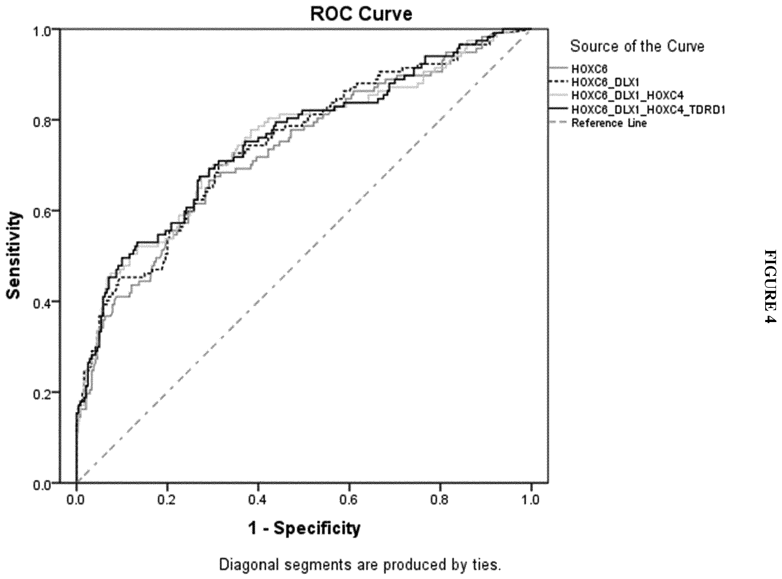

- **Figure 4**: shows the Receiver Operating Characteristic curve to visualize the prognostic potential of HOXC6, the combination of HOXC6 with DLX1, the combination of HOXC6 with DLX1 and HOXC4 and the combination of HOXC6 with DLX1 and HOXC4 and TDRD1 to discriminate Sign-PrCa from the Rest (i.e. negative biopsy and insignificant PCa) in the absence of an arbitrary cut-off value.

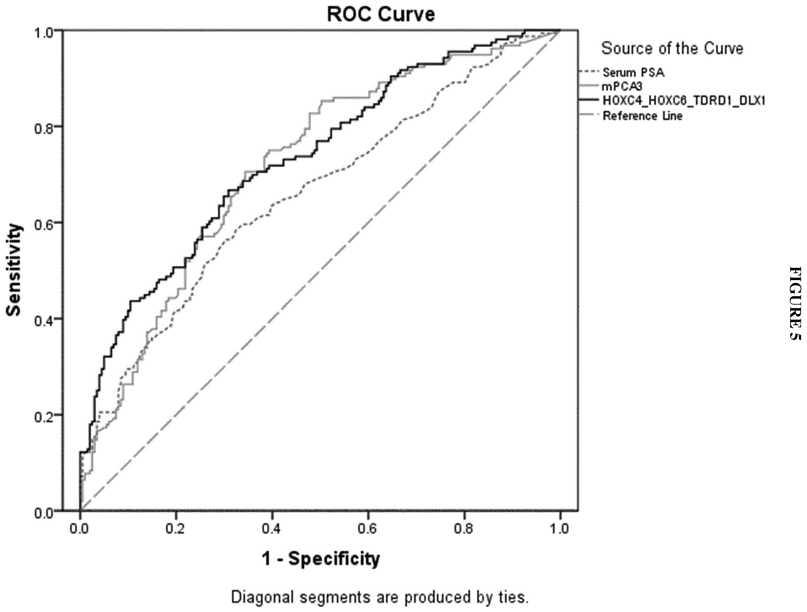

- **Figure 5**: shows the Receiver Operating Characteristic curve to visualize the diagnostic potential of the four gene panel (HOXC6, DLX1, HOXC4 and TDRD1) in comparison with mPCA3 levels and serum PSA values to discriminate PrCa_total from no PrCa in the absence of an arbitrary cut-off value.

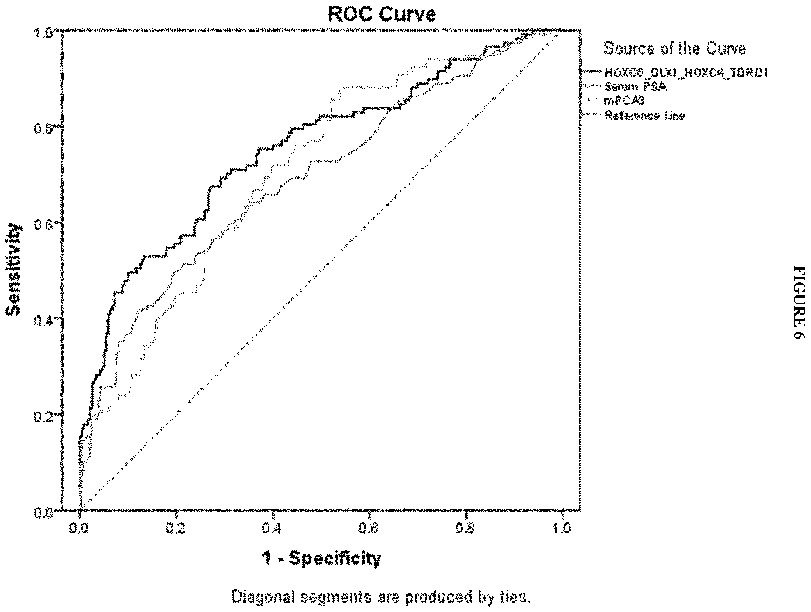

- **Figure 6**: shows the Receiver Operating Characteristic curve to visualize the prognostic potential of the four gene panel (HOXC6, DLX1, HOXC4 and TDRD1) in comparison with mPCA3 levels and serum PSA values to discriminate Sign-PrCa from the Rest (i.e. negative biopsy and insignificant PCa) in the absence of an arbitrary cut-off value.

### Example 1

### Initial preclinical discovery of candidate biomarkers

To identify markers for prostate cancer diagnosis and prediction of prognosis, the platform of Affymetrix GeneChips was used. Based on the robustness of this platform and the high resolution (many oligoprobes located in most exons of each gene) we decided to use the GeneChip Exon 1.0 ST array to determine the gene profiles of prostate tissue specimens that were collected at the Radboud University Nijmegen Medical Centre and Canisius Wilhemmina Hospital Nijmegen after a consent form approved by the institutional review board was signed by all participants.

Tissue specimens of patients with prostate cancer in the following groups were collected: normal prostate (NPr; n=8),Benign Prostatic Hyperplasia (BPH; n=12), Low grade prostate cancer (LG-PrCa; n=25),High grade prostate cancer (HG-PrCa;n=24), Castration resistant prostate cancer (CRPC; n=23)and prostate cancer metastases (PrCa Met; n=7).

Briefly, NPr tissue was obtained after radical or TURP from cancer free regions of these samples or from autopsy. BPH tissue was obtained from TURP or transvesical open prostatectomy (Hryntschak).LG-PrCa tissue was obtained from primary tumors with a Gleason Score ≤6, HG-PrCa tissue was obtained from primary tumors with a Gleason Score ≥7, CRPC tissue was obtained from patients that are progressive under endocrine therapy and who underwent a transurethral resection of the prostate (TURP) and PrCa Met tissue specimens were obtained from positive lymfnodes after LND or after autopsy. The tissues were snap frozen and cryostat sections were H.E. stained for classification by a pathologist.

Total RNA was extracted from tumor- and tumor-free areas using TRIzol (Invitrogen, Carlsbad, CA, USA) following manufacturer's instructions. The total RNA was DNase treated and purified with the Qiagen RNeasy mini kit (Qiagen, Valencia, CA, USA). Integrity of the RNA was checked by electrophoresis using the Agilent 2100 Bioanalyzer. Samples with RNA integrity number (RIN) >=6 were included.

Gene expression profiles were determined using the GeneChip Human Exon 1.0 Sense Target arrays (Affymetrix) according manufacturer's instructions. Gene-level and exon-level expression values were derived from the CEL file using the model-based Robust Multiarray Average algorithm as implemented in Partek® software (Partek Genomics Suite 6.6). P-values of differentially expressed genes between conditions were calculated using ANOVA analysis. For the identification of biomarkers the expression analysis of the different groups were compared: NP/BPH with LG- and HG-PCa, PCa-M+ with LG- and HG-PCa, CRPC with LG- and HG-PCa.

### Results and conclusion

Using the GeneChip Exon 1.0 ST array we were able to identify an initial group of 47 interesting genes. These selected genes were further analysed and validated with RT-qPCR technique using Taqman Low Density Arrays(TLDA; Applied Biosystems) as will be further elucidated in example 2.

### Example 2

### Preclinical selection of candidate biomarkers

Further analysis of the 47 biomarkers was performed using Taqman® Low Density Arrays (TLDA; Applied Biosystems). Tissue and urine specimens were collected at the Radboud University Nijmegen Medical Centre and Canisius Wilhemmina Hospital Nijmegen. Tissue specimens of patients with prostate cancer in the following groups were collected: normal prostate (NPr; n=6),Benign Prostatic Hyperplasia (BPH; n=6), Low grade prostate cancer (LG-PrCa; n=14),High grade prostate cancer (HG-PrCa;n=14), Castration resistant prostate cancer (CRPC; n=14)and prostate cancer metastases (PrCa Met; n=8). Tissue selection and RNA extraction and purification were performed as described in example 1. Two ug DNase-treated total RNA was reverse transcribed using Superscript II Reverse Transcriptase (Invitrogen) according manufacturer's instructions.

For the validation not only prostate tissue specimens were used. To investigate whether the selected markers could successfully be detected in body fluids also normal bladder tissue specimens (n=2), peripheral blood lymphocytes (PBL,n=2) and urinary sediment specimens from patients which had PrCa in their biopsies (n=9) and 7 from patients with negative biopsies (n=7)) were included included in the marker validation step. The background signal of the markers in normal bladder and urinary sediments from patients without prostate cancer should be low.

First voided urine samples were collected after digital rectal examination (DRE) from men scheduled for prostate cancer. After urine specimen collection, the urologist performed prostate biopsies according to a standard protocol. Prostate biopsies were evaluated and in case prostate cancer was present the Gleason score was determined. First voided urine after DRE (20-30 ml) was collected in a tube containing 2 ml 0.5M EDTA pH 8.0. All samples were immediately cooled to 4°C and were mailed 10 in batches with cold packs to the laboratory.

The samples were processed within 48 h after the samples were acquired to guarantee good sample quality. Upon centrifugation at 4°C and 1,800 x g for 10 minutes, urinary sediments were obtained. These urinary sediments were washed twice with ice-cold buffered sodium chloride solution (at 4°C and 1,800 x g for 10 minutes), snap-frozen in liquid nitrogen, and stored at -70°C. Total RNA was extracted from these urinary sediments, using Trizol according to the manufacturers protocol. Two additional steps were added. Firstly, 2 µl glycogen (15 mg/ml) was added as a carrier (Ambion, Austin (TX), USA) before precipitation with isopropanol. Secondly, a second precipitation step with 3M sodium-acetate pH 5.2 and 100% ethanol was performed to discard traces of Trizol.

The RNA was DNase treated using amplification grade DNaseI (Invitrogen™, Breda, the Netherlands) according to the manufacturers protocol. Again glycogen was added as carrier and the RNA was precipitated with 3M sodium-acetate pH 5.2 and 100% ethanol for 2hr at -20°C. The RNA was dissolved in 16.5 µl RNase-free water and 1 µg of total RNA was used for RNA amplification using the Ambion® WT Expression Kit (Ambion, Austin (TX), USA) according to the manufacturer's instructions. Two ug amplified RNA was reverse transcribed using Superscript II Reverse Transcriptase (Invitrogen) according manufacturer's instructions.

To determine gene expressions levels the cDNA generated from RNA extracted from both tissue specimens and urinary sediments was used as template in the TLDA's. After centrifugation of the 384-well TLDA cards for 1 minute at 280g the cards were run in a 7900 HT Fast Real-Time PCR System (Applied Biosystems). Raw data were recorded with the Sequence detection System (SDS) software of the instruments analyzed with RQ documents and the RQ Manager Software for automated data analysis. Delta cycle threshold 30 (Ct) values were determined as the difference between the Ct of each test gene and the Ct of hypoxanthine phosphoribosyltransferase 1 (HPRT1) (endogenous control gene). Furthermore, gene expression values were calculated based on the comparative threshold cycle (Ct) method, in which a normal prostate RNA sample was designated as a calibrator to which the other samples were compared.

### Results

After analysis of the generated data, a list of 10 most promising biomarkers indicative for prostate cancer and the prognosis thereof was obtained.

### Example 3

### Final selection and model development of candidate biomarkers and combinations thereof in a clinical prospective study

### Study design

In a prospective multicenter study it was tested whether the ten in the pre-clinical biomarker discovery selected biomarkers or a combination of these markers could identify patients with PrCa_total or Sign-PrCa based on expression levels in urine samples. If so, these markers or a combination of markers could be used in an *in vitro* non-invasive method for diagnosing PrCa_total or Sign-PrCa.

Men who were scheduled for (initial or repeat) prostate biopsies, based on elevated sPSA levels, a family history of PCa or an abnormal DRE were included. First-catch urine after DRE was collected from 443 men. Prostate biopsies were performed and evaluated per hospital's standard procedure. In addition, one experienced genitourinary pathologist reviewed all biopsy Gleason scores independently, being blinded for the biomarker scores. Men were recruited at six urology clinics in the Netherlands (Radboud University Nijmegen Medical Centre, Nijmegen; Academic Medical Centre, Amsterdam; ZGT Hospital, Hengelo; Canisius Wilhelmina Hospital, Nijmegen; Scheper Hospital, Emmen; and St. Elisabeth Hospital, Tilburg). Exclusion criteria were: history of PCa, medical therapy known to affect sPSA levels, prostate biopsies within three months prior to enrolment, or invasive treatment for BPH within six months prior to enrolment. The respective independent ethics committees approved the study protocol and all included patients provided written informed consent. The biomarker discovery and the clinical validation study were both performed in accordance with the STARD (STAndards for Reporting of Diagnostic accuracy) criteria and REMARK (Reporting Recommendations for Tumor Marker Prognostic Studies) guidelines.

### Data collection

Clinical pathological data were collected for each patient, including: age, sPSA, DRE and TRUS results, prostate volume, biopsy results (current and history), radiological results, clinical TNM stage (if diagnosed with PCa) and radical prostatectomy results (if applicable).

### Specimen processing

First-catch urine specimens after DRE were processed using a validated standard operating procedure (SOP), total RNA was extracted from the urinary sediments, RNA was amplified and cDNA was generated as was described in example 2.

To determine the expression levels (copy numbers) for the selected biomarkers and for the genes KLK3, PCA3 and HPRT1 in these specimens, optimized real-time quantitative PCR assays were developed (according the MIQE guidelines). Fluorescence based real-time PCR assays with primers and hydrolysis probe were designed. PCR products were cloned into vectors and calibration curves with a wide linear dynamic range (10 - 1.000.000 copies) were made in order to calculate copy numbers.

Two µl of each cDNA sample was amplified in a 20 µl PCR reaction containing optimized amounts of forward primer and reverse primer, 2 pmol of hydrolysis probe and 1x Probes Master mix (Roche). The following amplification conditions were used: 95°C for 10 minutes followed by 50 cycles at 95°C for 10 seconds, 60°C for 30 seconds and a final cooling step at 40°C for 55 seconds (LightCycler LC480, Roche). The crossing point (Cp) values were determined using the Lightcycler 480 SW 1.5 software (Roche). The Cp values of the samples were converted to copy numbers by interpolation in the generated calibration curve. Samples that had *HPRT1* mRNA < 4000 copies were excluded for this study.

### Statistical analyses

Statistical analyses were performed with SPSS® version 20.0. Two-sided P values of ≤ 0.05 were considered to indicate statistical significance. For a normal distribution expression data were log transformed and an univariate analysis was performed using forward logistic regression to determine whether the single biomarkers had significant predictive value (p<0.05) for diagnosis of PrCa_total and/or Sign-PrCa. The Odds Ratio's (O.R.) and corresponding 95% Confidence Intervals (CI) were determined.

To determine whether the markers had independent predictive value and had additional value to each other a multivariate analysis was performed using forward logistic regression. The best combinations of biomarkers for the prediction of PrCa or Sign-PrCa were identified.

To visualize the performance of the selected biomarkers in the absence of an arbitrary cut-off value, the data were summarized using a Receiver Operating Characteristic (ROC) curve. In a ROC curve, the true positive rate to detect prostate cancer (Sensitivity) is plotted in function of the false positive rate (*i.e.* positives in the no-PrCa group) (1-Specificity) for different cut-off points of a parameter. Each point on the ROC curve represents a sensitivity/specificity pair corresponding to a particular decision threshold. The area under the ROC curve is a measure of how well a parameter can distinguish between two groups, *e.g.* (PrCa_total versus no-PrCa). When the variable under study cannot distinguish between the two groups, *i.e.* in case there is no difference between the two distributions, the area will be equal to 0.5 (the ROC curve will coincide with the diagonal). A test with perfect discrimination (no overlap in the two distributions) has a ROC curve that passes through the upper left corner (100% sensitivity, 100% specificity). Therefore the closer the ROC curve is to the upper left corner, the higher the overall accuracy of the test.

### Results

In total, 443 patients were enrolled in this prospective multicenter study. Samples with HPRT1 mRNA < 4000 copies were excluded (n=85). This resulted in 358 evaluable samples. In this cohort, 157 patients had prostate cancer in their biopsies. Of all the prostate cancers found (PrCa_total), 93 (59%) had a Gleason score >=7 and 64 (41%) had a Gleason score <=6. Furthermore, of all the prostate cancers found (PrCa_total), 118 (75%) could be classified as significant prostate cancer (Sign-PrCa), i.e. a Gleason Score of >=7 and/or a percentage biopsy positive cores >=33% and/or a clinical stage >=T2 (Epstein criteria)

**Table 1A** shows the univariate forward logistic regression data with the 10 biomarkers for diagnosis of PrCa. The P-values and Odds Ratios (O.R.) with corresponding 95% Confidence Intervals (CI) are presented. mRNA levels of HOXC4, HOXC6, DLX1, TDRD1, NKAIN1, PTPRT, CGREF1, GLYATL1 and PPFIA2 were significantly higher in patients with PrCa (PrCa_total) compared to patients without PrCa, whereas RRM2 was not.

**Table 1A: Univariate forward logistic regression data with the 10 biomarkers for diagnosis of PrCa.**

| | P-value | OR | 95% C.I. | |
|---|---|---|---|---|
| | | | Lower | Upper |
| LN_HOXC4 | 8.4E-06 | 1.35 | 1.18 | 1.54 |
| LN_HOXC6 | 1.7E-10 | 1.60 | 1.39 | 1.85 |
| LN_DLX1 | 4.7E-09 | 1.31 | 1.19 | 1.43 |
| LN_TDRD1 | 3.2E-07 | 1.21 | 1.12 | 1.30 |
| LN_NKAIN1 | 2.9E-04 | 1.21 | 1.09 | 1.34 |
| LN_PTPRT | 6.9E-06 | 1.19 | 1.10 | 1.28 |
| LN_RRM2 | 2.4E-01 | 1.11 | 0.94 | 1.31 |
| LN_CGREF1 | 3.0E-03 | 1.19 | 1.06 | 1.33 |
| LN_GLYATL1 | 2.0E-03 | 1.30 | 1.10 | 1.55 |
| LN_PPFIA2 | 2.5E-05 | 1.18 | 1.09 | 1.27 |

**Table 1B** shows the univariate forward logistic regression data with the 10 biomarkers for diagnosis of Sign-PrCa. mRNA levels of HOXC4, HOXC6, DLX1, TDRD1, NKAIN1, PTPRT, CGREF1, GLYATL1 and PPFIA2 were significantly higher in patients with Sign-PrCa compared to the rest (patients without PrCa or insignificant PrCa), whereas RRM2 was not.

**Table 1B: Univariate forward logistic regression data with the 10 biomarkers for diagnosis of Sign-PrCa.**

| | P-value | OR | 95% C.I. | |
|---|---|---|---|---|
| | | | Lower | Upper |
| LN_HOXC4 | 6.7E-05 | 1.34 | 1.16 | 1.55 |
| LN_HOXC6 | 4.7E-10 | 1.65 | 1.41 | 1.94 |
| LN_DLX1 | 1.3E-11 | 1.36 | 1.24 | 1.48 |
| LN_TDRD1 | 1.0E-09 | 1.31 | 1.20 | 1.43 |
| LN_NKAIN1 | 6.1E-05 | 1.26 | 1.13 | 1.42 |
| LN_PTPRT | 4.8E-08 | 1.24 | 1.15 | 1.34 |
| LN_RRM2 | 5.9E-02 | 1.19 | 0.99 | 1.42 |
| LN_CGREF1 | 2.4E-04 | 1.28 | 1.12 | 1.47 |
| LN_GLYATL1 | 5.0E-03 | 1.30 | 1.08 | 1.56 |
| LN_PPFIA2 | 2.4E-05 | 1.20 | 1.10 | 1.30 |

**Table 2A** shows the multivariate analysis data with forward logistic regression for diagnosis of PrCa. Only DLX1 and HOXC6 had independent additional predictive value for diagnosing PrCa (P-value <0.05) and are stepwise added to the model.

**Table 2A: Multivariate analysis data with forward logistic regression for diagnosis of PrCa.**

| **Variables in the Equation** | | | | | |
|---|---|---|---|---|---|
| | | P-value | O.R. | 95% C.I. | |
| | | | | Lower | Upper |
| Step 1 | LN_HOXC6 | <0.001 | 1.61 | 1.39 | 1.86 |
| | Constant | <0.001 | 0.04 | | |
| Step 2 | LN_HOXC6 | <0.001 | 1.47 | 1.26 | 1.71 |
| | LN_DLX1 | <0.001 | 1.21 | 1.105 | 1.34 |
| | Constant | <0.001 | 0.05 | | |

| **Variables not in the Equation** | | | | | |
|---|---|---|---|---|---|
| | | | | P-value | |
| Step 2 | | LN_HOXC4 | | 0.40 | |
| | | LN_TDRD1 | | 0.20 | |
| | | LN_PPFIA | | 0.40 | |
| | | LN_GLYATL1 | | 0.33 | |
| | | LN_RRM2 | | 0.20 | |
| | | LN_CGREF1 | | 0.06 | |
| | | LN_NKAIN1 | | 0.60 | |
| | | LN_PTPRT | | 0.72 | |
| | | LN_RRM2 | | 0.20 | |

**Table 2B** shows the multivariate analysis data with forward logistic regression for diagnosis of Sign-PrCa. Only DLX1, HOXC6 and TDRD1 had independent additional predictive value for diagnosing Sign-PrCa (P-value <0.05) and are stepwise added to the model.

**Table 2B: multivariate analysis data with forward logistic regression for diagnosis of Sign-PrCa.**

| **Variables in the Equation** | | | | | |
|---|---|---|---|---|---|
| | | P-value | OR | 95% C.I. | |
| | | | | Lower | Upper |
| Step 1 | LN_DLX1 | <0.001 | 1.37 | 1.25 | 1.49 |
| | Constant | <0.001 | 0.27 | | |
| Step 2 | LN_HOXC6 | <0.001 | 1.43 | 1.21 | 1.69 |
| | LN_DLX1 | <0.001 | 1.26 | 1.14 | 1.40 |
| | Constant | <0.001 | 0.03 | | |
| Step 3 | LN_HOXC6 | <0.001 | 1.36 | 1.15 | 1.62 |
| | LN_DLX1 | 0,001 | 1.20 | 1.08 | 1.34 |
| | LN_TDRD1 | 0,013 | 1.14 | 1.03 | 1.26 |
| | Constant | <0.001 | 0.03 | | |

| **Variables not in the Equation** | | | | | |
|---|---|---|---|---|---|
| | | | | P-value | |
| Step 3 | | LN_HOXC4 | | 0.75 | |
| | | LN_GLYATL1 | | 0.18 | |
| | | LN_NKAIN1 | | 0.44 | |
| | | LN_PPFIA2 | | 0.72 | |
| | | LN_PTPRT | | 0.49 | |
| | | LN_RRM2 | | 0.15 | |
| | | LN_CGREF1 | | 0.10 | |

HOXC4 and HOXC6 are transcribed from the same transcriptional unit. The expression patterns of both genes show high correlation and both are significant diagnostic and prognostic biomarkers. In the multivariate logistic regression with the 10 biomarkers and with both genes included HOXC4 is not an independent predictor. HOXC6 performs better for diagnosing PrCa_total and Sign-PrCa than HOXC4 however, the differences are very small and these markers can be interexchanged in many cases. When a multivariate analysis is performed without either HOXC4 or HOXC6, of all other 8 biomarkers again only DLX1 and/or TDRD1 have independent additional value and are added to the model for diagnosing PrCa_total and Sign-PrCa. Therefore it was decided to perform further detailed data analysis for the four genes HOXC4, HOXC6, DLX1 and TDRD1 and combinations thereof.

In **Tables 3A** and **3B,** the copy numbers, copy number ranges and fold-changes between the groups of the individual genes of interest are shown for no PrCa, and PrCa_total (diagnosis) and for Sign-PrCa and the rest (prognosis) for the cohort of 358 urinary sediments.

**Table 3A: The copy numbers, copy number ranges and fold-changes between the groups of the individual genes.**

| | PrCa | | no PrCa | | Fold |
|---|---|---|---|---|---|
| | n=157 | | n=201 | | Change |
| | copies | Range | copies | Range | PrCa/no PrCa |
| HOXC4 | 25288 | (1-355000) | 8888 | (1-116000) | 2.9 |
| HOXC6 | 5433 | (1-193000) | 729 | (1-8970) | 7.5 |
| TDRD1 | 17629 | (1-727000) | 619 | (1-56400) | 28.5 |
| DLX1 | 1510 | (1-66700) | 42 | (1-3690) | 36.2 |

**Table 3B:**

| | Sign PrCa | | Rest* | | Fold |
|---|---|---|---|---|---|
| | n=118 | | n=240 | | Change |
| | copies | Range | copies | Range | Sign PrCa/Rest |
| HOXC4 | 29843 | (1-355000) | 9313 | (1-116000) | 3.2 |
| HOXC6 | 6873 | (1-193000) | 785 | (1-8970) | 8.8 |
| TDRD1 | 23363 | (1-727000) | 587 | (1-56400) | 39.8 |
| DLX1 | 1996 | (1-66700) | 42 | (1-3690) | 47.7 |

| | | | | | |
|---|---|---|---|---|---|
| Rest*= neg. biopsy and insign PrCa (GS<7, biopsy pos. cores <33% and < T2 stage) | | | | | |

To visualize the performance of the four selected biomarkers to discriminate PrCa_total from no-PrCa and to discriminate Sign-PrCa from the rest (*i.e.* negative biopsy and insignificant PrCa) the data were summarized using a Receiver Operating Characteristic (ROC) curve. The ROC curves were made for the four single biomarkers and for the combinations of these biomarkers. In a Receiver under Operation (ROC)-curve, the diagnostic potential of HOXC4, HOXC6, DLX1 and TDRD1 expression in the cohort of 358 urinary sediments to discriminate no PrCa from PrCa_total is visualized in **Figure 1****.** The area under the curve (AUC) for HOXC4 is 0.69 (95% CI: 0.63-0.74), for HOXC6 is 0.72 (95% CI: 0.67-0.77), for DLX1 is 0.65 (95%CI: 0.59-0.71) and for TDRD1 is 0.66 (95% CI: 0.60-0.72).

In a Receiver under Operation (ROC)-curve, the prognostic potential of HOXC4, HOXC6, DLX1 and TDRD1 expression in urinary sediments to discriminate Sign-PrCa from the rest (i.e. insignificant PrCa and negative biopsies) is visualized in **Figure 2****.** The area under the curve (AUC) for HOXC4 is 0.69 (95% CI: 0.63-0.75), for HOXC6 is 0.73 (95% CI:0.67-0.79), for DLX1 is 0.68 (95% CI: 0.62-0.74) and for TDRD1 is 0.71 (95% CI: 0.65-0.77).

The diagnostic potential of combinations of HOXC4, HOXC6, DLX1 and TDRD1 for the expression in the cohort of 358 urinary sediments to discriminate no PrCa from PrCa_total is visualized in a Receiver under Operation (ROC)-curve in **Figure 3****.** The area under the curve (AUC) for HOXC6 expression is 0.719 (95% CI: 0.67-0.77), for the combination of HOXC6 with DLX1 is 0.726 (95% CI:0.67-0.78), for the combination of HOXC6, DLX1 and HOXC4 expression is 0.733 (95% CI: 0.68-0.79) and for the combination of HOXC6, DLX1, HOXC4 and TDRD1 is 0.732 (95% CI: 0.68-0.78).

To discriminate no PrCa from PrCa_total (diagnosis) in urinary sediments, at a specificities of 70%, 80% and 90% cut-off values were extrapolated from the ROC-curves and the sensitivity, positive predictive value (PPV), negative predictive value (NPV) was calculated for HOXC6 and for the combinations with HOXC4, DLX1 and TDRD1. Furthermore the number of patients with prostate cancer detected by the markers was determined as well. The results are summarized in **Table 4A.**

**Table 4A: Discrimination of no PrCafrom PrCa_total (diagnosis) in urinary sediments**

| | AUC | Spec. 70% | | | | Spec. 80% | | | | Spec. 90% | | | |
|---|---|---|---|---|---|---|---|---|---|---|---|---|---|
| | | Sens. | PPV | NPV | PCa | Sens. | PPV | NPV | PCa | Sens. | PPV | NPV | PCa |
| HOXC6 | 0,719 | 62 | 62 | 70 | 97 | 46 | 65 | 66 | 73 | 34 | 73 | 64 | 53 |
| HOXC6/DLX1 | 0,726 | 62 | 62 | 70 | 97 | 48 | 65 | 66 | 75 | 38 | 75 | 65 | 59 |
| HOXC6/HOXC4/DLX1 | 0,733 | 64 | 63 | 71 | 100 | 50 | 66 | 67 | 78 | 42 | 77 | 67 | 66 |
| HOXC6/HOXC4/DLX1/TDRD1 | 0,732 | 65 | 63 | 72 | 102 | 51 | 66 | 68 | 79 | 41 | 76 | 66 | 64 |
| HOXC4/TDRD1/DLX1 | 0,722 | 63 | 62 | 71 | 98 | 53 | 67 | 69 | 83 | 39 | 75 | 66 | 61 |
| serum PSA | 0,658 | 55 | 59 | 67 | 87 | 41 | 62 | 64 | 65 | 29 | 70 | 62 | 46 |
| mPCA3 | 0,716 | 62 | 62 | 70 | 97 | 44 | 63 | 65 | 69 | 26 | 67 | 61 | 41 |

| | | | | | | | | | | | | | |
|---|---|---|---|---|---|---|---|---|---|---|---|---|---|
| PCa = number of detected cancer patients | | | | | | | | | | | | | |

The prognostic potential of combinations of HOXC4, HOXC6, DLX1 and TDRD1 for the expression in the cohort of 358 urinary sediments to discriminate Sign-PrCa from the rest (insignificant PrCa and negative biopsies)are visualized in a Receiver under Operation (ROC)-curve in **Figure 4****.** The area under the curve (AUC) for HOXC6 expression is 0.731 (95% CI: 0.67-0.79), for the combination of HOXC6 with DLX1 is 0.745 (95% CI:0.69-0.80), for the combination of HOXC6, DLX1 and HOXC4 is 0.750 (95% CI: 0.69-0.81) and for the combination of HOXC6, DLX1, HOXC4 and TDRD1 expression is 0.753 (95% CI: 0.70-0.81).

To discriminate Sign-PrCa from the rest (prognosis) in urinary sediments, at a specificities of 70%, 80% and 90% cut-off values were extrapolated from the ROC-curves and the sensitivity, positive predictive value (PPV), negative predictive value (NPV) were calculated for HOXC6 and for the combinations with HOXC4, DLX1 and TDRD1. Furthermore the number of significant prostate cancers detected was determined as well. The results are summarized in **Table 4B.**

**Table 4B: Discrimination of Sign-PrCa from the rest (prognosis) in urinary sediments**

| | AUC | Spec. 70% | | | | Spec. 80% | | | | Spec. 90% | | | |
|---|---|---|---|---|---|---|---|---|---|---|---|---|---|
| | | Sens. | PPV | NPV | PCa | Sens. | PPV | NPV | PCa | Sens. | PPV | NPV | PCa |
| HOXC6 | 0,731 | 66 | 52 | 81 | 78 | 53 | 57 | 78 | 63 | 41 | 67 | 76 | 48 |
| HOXC6/DLX1 | 0,745 | 65 | 52 | 80 | 77 | 53 | 57 | 78 | 63 | 45 | 69 | 77 | 53 |
| HOXC6/HOXC4/DLX1 | 0,750 | 70 | 54 | 83 | 83 | 54 | 57 | 78 | 64 | 47 | 70 | 77 | 55 |
| HOXC6/HOXC4/DLX1/TDRD1 | 0,753 | 69 | 53 | 82 | 81 | 56 | 58 | 79 | 65 | 50 | 71 | 79 | 58 |
| HOXC4/TDRD1/DLX1 | 0,749 | 70 | 53 | 83 | 82 | 58 | 59 | 80 | 68 | 43 | 68 | 76 | 50 |
| serum PSA | 0,693 | 57 | 49 | 77 | 68 | 49 | 55 | 76 | 58 | 36 | 63 | 74 | 43 |
| mPCA3 | 0,706 | 59 | 49 | 77 | 69 | 44 | 52 | 74 | 52 | 25 | 55 | 71 | 29 |

| | | | | | | | | | | | | | |
|---|---|---|---|---|---|---|---|---|---|---|---|---|---|
| PCa = number of detected cancer patients | | | | | | | | | | | | | |

In a Receiver under Operation (ROC)-curve, the diagnostic potential of the combination of HOXC6, DLX1, HOXC4 and TDRD1 expression and the expression of PCA3 in urinary sediments and the serum PSA value to discriminate no PrCa from PrCa_total is visualized in **Figure 5****.** The area under curve (AUC) for the combination of HOXC6, DLX1,HOXC4 and TDRD1 expression is 0.732 (95% CI: 0.68-0.78), for the expression of PCA3 is 0,716 (95% CI: 0.66-0.77) and for serum PSA is 0,658 (95% CI: 0.60-0.72).

In a Receiver under Operation (ROC)-curve, the prognostic potential of the combination of HOXC6 with DLX1, HOXC4 and TDRD1 expression, the expression of PCA3 in urinary sediments and the serum PSA value to discriminate Sign-PrCa from the rest (insignificant PrCa and negative biopsies) is visualized in **Figure 6****.** The area under curve (AUC) for the combination of HOXC6, DLX1, HOXC4 and TDRD1 expression is 0.753 (95% CI: 0.70-0.81), for the expression of PCA3 is 0,706 (95% CI: 0.65-0.76) and for serum PSA is 0,693 (95% CI: 0.63-0.75).

### Discussion

The present example shows that the individual selected genes (HOXC4, HOXC6, DLX1 and TDRD1) each show overexpression in prostate cancer versus no-prostate cancer (diagnosis) and overexpression in Sign-PrCa versus the rest (prognosis). Especially TDRD1 and DLX1 show in both situations a high fold change difference between the groups.

Based on the AUC of the ROC curves for the single biomarkers, HOXC6 shows the best results for diagnosing prostate cancer (PrCa_total) (**Figure 1**). When combined with DLX1, HOXC4 and or TDRD1 the overall diagnostic performance is improved. This does not mean that this combination is the best under all conditions. Depending on where the biomarkers will be used for (screening, reduction of biopsies) a high specificity or a high sensitivity is desired. If a higher sensitivity is desired, e.g. at a lower specificity of 80%, the combination of HOXC4, DLX1 and TDRD1 performs best (see **Table 4A**).

At a specificity of 70%, HOXC6 detects 97 of the prostate cancers (sensitivity of 62%), when combined with HOXC4, DLX1 and TDRD1 102 prostate cancers are detected (sensitivity of 65%). If a higher specificity is required, e.g. at 80%, the combination of HOXC4, DLX1 and TDRD1 performs best; 83 cancers are detected (sensitivity 53%) and this is a significant improvement compared to the number of cancers detected by serum PSA (65) and mPCA3 (69). These tests have a sensitivity of only 41% and 44% respectively at a specificity of 80% (**Table 4A**).

For the detection of significant prostate cancer (prognosis), from the 4 single biomarkers HOXC6 shows the best results based on the AUC of the ROC curves (**Figure 6**).

When combined with DLX1, HOXC4 and or TDRD1 the prognostic performance is improved, especially at a high specificity.

At a specificity of 90%, HOXC6 detects 48 of the significant prostate cancers (sensitivity of 41%), when combined with HOXC4, DLX1 and TDRD1 58 significant prostate cancers are detected (sensitivity of 50%). This is a significant improvement compared to the number of cancers detected by serum PSA (43) and mPCA3 (29). These tests have a sensitivity of only 36% and 25% respectively at a specificity of 90% (**Table 4B**).

For a urologist the probability of having significant prostate cancer should be as high as possible. Therefore the specificity and PPV are very important for treatment decision making. At a high specificity of 90%, the PPV of the four gene panel is 71%. This indicates that a man with a positive test for the four gene panel has a probability of 71% for having significant prostate cancer. This is significantly higher than the PPV for serum PSA (63%) and mPCA3 (55%).

### Conclusion

As demonstrated above, the present molecular markers, or biomarkers, for prostate cancer provide, in combination, methods and means allowing discrimination between prostate cancer and no-prostate cancer and allowing detecting significant prostate cancer from the rest (insignificant PrCa and negative biopsies) with improved sensitivity, specificity, PPV and NPV, especially when compared with presently available biomarkers such as the serum PSA and mPCA3.

## Claims

1. Method for *in vitro* establishing prostate cancer (PrCa) in a sample originating from a human individual suspected of or suffering from prostate cancer comprising:
a) determining expression levels of DLX1 and HOXC6;
b) establishing up-regulation of the expression levels of DLX1 and HOXC6 as compared to expression levels of DLX1 and HOXC6 in a sample originating from an individual not suffering from prostate cancer or as compared to a reference value indicative of a non-disease expression level; and
c) establishing the presence or absence of prostate cancer (PrCa) based on the up-regulation of the expression levels of DLX1 and HOXC6 in said sample.
wherein step (a) further comprises determining the expression levels of HOXC4 and TDRD1; wherein step (b) further comprises establishing up-regulation of the expression levels of HOXC4 and TDRD1 as compared to expression levels of HOXC4 and TDRD1 in a sample originating from an individual not suffering from prostate cancer or as compared to a reference value indicative of a non-disease expression level; and wherein step (c) comprises establishing the presence or absence of prostate cancer (PrCa) based on the up-regulation of the expression levels of DLX1, HOXC6, HOXC4 and TDRD1 in said sample.

2. Method according to claim 1, wherein establishing prostate cancer (PrCa) comprises establishing high grade (Gleason score >= 7) or low grade (Gleason score <7) prostate cancer.

3. Method according to any of the claim 1 or claim 2, wherein establishing prostate cancer (PrCa) comprises establishing a percentage of positive cores of >=33%.

4. Method according to claim 1, wherein establishing prostate cancer (PrCa) comprises establishing a clinical stage of >= T2 according to the Epstein criteria.

5. Method according to any of the claims 1 to 4, wherein establishing prostate cancer (PrCa) comprises establishing high grade (Gleason score >= 7) prostate cancer, establishing a percentage of positive cores of >=33% and establishing a clinical stage of >= T2 according to the Epstein criteria.

6. Method according to any of the claims 1 to 5, wherein determining expression levels comprises determining mRNA expression levels and/or wherein determining expression levels comprises determining protein levels.

7. Method according to any of the claims 1 to 6, wherein said sample is a sample selected from the group consisting of urine, urine derived, prostatic fluid, prostatic fluid derived, ejaculate and ejaculate derived.

8. Use of a combination of a DLX1 expression marker, a HOXC6 expression marker, a HOXC4 expression marker and a TDRD1 expression marker for *in vitro* establishing prostate cancer (PrCa).

9. Use according to claim 8, wherein establishing prostate cancer comprises establishing prostate cancer in a sample selected from the group consisting of urine, urine derived, prostatic fluid, prostatic fluid derived, ejaculate and ejaculate derived.

10. Use of a kit of parts in a method according to any one of the claims 1 to 7 for *in vitro* establishing prostate cancer (PrCa) in a sample originating from a human individual suspected of or suffering from prostate cancer, the kit comprises:
- expression level analysis means for determining the expression levels of HOXC6 and DLX1;
- instructions for use.
the kit further comprising expression level analysis means for determining the expression levels of HOXC4 and TDRD1.

## Patentansprüche

1. Verfahren für den In-vitro-Nachweis von Prostatakrebs (PrCa) in einer Probe, die von einem menschlichen Individuum stammt, für das der Verdacht besteht, dass es unter Prostatakrebs leidet, das aufweist:
a) Bestimmen von Expressionsniveaus von DLX1 und HOXC6;
b) Nachweisen der Hoch-Regulation der Expressionsniveaus von DLX1 und HOXC6 im Vergleich zu Expressionsniveaus von DLX1 und HOXC6 in einer Probe, die von einem Individuum stammt, das nicht unter Prostatakrebs leidet, oder im Vergleich zu einem Referenzwert, der ein nicht krankes Expressionsniveau anzeigt; und
c) Nachweisen des Vorhandenseins oder Nichtvorhandenseins von Prostatakrebs (PrCa) basierend auf der Hoch-Regulation der Expressionsniveaus von DLX1 und HOXC6 in der Probe,
wobei der Schritt (a) ferner die Bestimmung der Expressionsniveaus von HOXC4 und TDRD1 aufweist; wobei der Schritt (b) ferner den Nachweis der Hoch-Regulation der Expressionsniveaus von HOXC4 und TDRD1 aufweist im Vergleich zu Expressionsniveaus von HOXC4 und TDRD1 in einer Probe, die von einem Individuum stammt, das nicht unter Prostatakrebs leidet, oder im Vergleich zu einem Referenzwert, der ein nicht krankes Expressionsniveau anzeigt; und wobei der Schritt (c) den Nachweis des Vorhandenseins oder Nichtvorhandenseins von Prostatakrebs (PrCa) basierend auf der Hoch-Regulation der Expressionsniveaus von DLX1, HOXC6, HOXC4 und TDRD1 in der Probe aufweist.

2. Verfahren nach Anspruch 1, wobei der Nachweis von Prostatakrebs (PrCa) den Nachweis von fortgeschrittenem (Gleason-Score >= 7) oder niedriggradigem (Gleason-Score < 7) Prostatakrebs aufweist.

3. Verfahren nach Anspruch 1 oder Anspruch 2, wobei der Nachweis von Prostatakrebs (PrCa) den Nachweis eines Prozentsatzes positiver Kerne >= 33% aufweist.

4. Verfahren nach Anspruch 1, wobei der Nachweis von Prostatakrebs (PrCa) den Nachweis eines klinischen Stadiums >= T2 gemäß den Epstein-Kriterien aufweist.

5. Verfahren nach einem der Ansprüche 1 bis 4, wobei der Nachweis von Prostatakrebs (PrCa) den Nachweis von fortgeschrittenem (Gleason-Score >= 7), den Nachweis eines Prozentsatzes positiver Kerne >= 33% und den Nachweis eines klinischen Stadiums >= T2 gemäß den Epstein-Kriterien aufweist.

6. Verfahren nach einem der Ansprüche 1 bis 5, wobei die Bestimmung von Expressionsniveaus die Bestimmung von mRNA-Expressionsniveaus aufweist und/oder wobei die Bestimmung von Expressionsniveaus die Bestimmung von Proteinspiegeln aufweist.

7. Verfahren nach einem der Ansprüche 1 bis 6, wobei die Probe eine Probe ist, die aus der Gruppe ausgewählt wird, die besteht aus: Urin, aus Urin gewonnen, Prostataflüssigkeit, aus Prostataflüssigkeit gewonnen, Ejakulat und aus Ejakulat gewonnen.

8. Verwendung einer Kombination eines DLX1-Expressionsmarkers, eines HOXC6-Expressionsmarkers, eines HOXC4-Expressionsmarkers und eines TDRD1-Expressionsmarkers für den In-vitro-Nachweis von Prostatakrebs (PrCa).

9. Verwendung nach Anspruch 8, wobei der Nachweis von Prostatakrebs den Nachweis von Prostatakrebs in einer Probe aufweist, die aus einer Gruppe ausgewählt wird, die besteht aus: Urin, aus Urin gewonnen, Prostataflüssigkeit, aus Prostataflüssigkeit gewonnen, Ejakulat und aus Ejakulat gewonnen.

10. Verwendung eines Teilesatzes in einem Verfahren nach einem der Ansprüche 1 bis 7 zum In-vitro-Nachweis von Prostatakrebs (PrCa) in einer Probe, die von einem menschlichen Individuum stammt, für das der Verdacht besteht, dass es unter Prostatakrebs leidet, wobei der Satz aufweist:
- Einrichtungen zur Expressionsniveau-Analyse zur Bestimmung der Expressionsniveaus von HOXC6 und DLX1;
- Gebrauchsanweisung,
wobei der Satz ferner Einrichtungen zur Expressionsniveau-Analyse zur Bestimmung der Expressionsniveaus von HOXC4 und TDRD1 aufweist.

## Revendications

1. Procédé pour l'établissement *in vitro* d'un cancer de la prostate (CaP) dans un échantillon provenant d'un individu humain suspecté de ou souffrant d'un cancer de la prostate comprenant :
a) la détermination des taux d'expression de DLX1 et HOXC6 ;
b) l'établissement d'une régulation positive des taux d'expression de DLX1 et HOXC6 comparativement aux taux d'expression de DLX1 et HOXC6 dans un échantillon provenant d'un individu ne souffrant pas d'un cancer de la prostate ou comparativement à une valeur de référence indicative d'un taux d'expression non pathologique ; et
c) l'établissement de la présence ou de l'absence d'un cancer de la prostate (CaP) en se basant sur la régulation positive des taux d'expression de DLX1 et HOXC6 dans ledit échantillon,
dans lequel l'étape (a) comprend en outre la détermination des taux d'expression de HOXC4 et TDRD1 ;
dans lequel l'étape (b) comprend en outre l'établissement de la régulation positive des taux d'expression de HOXC4 et TDRD1 comparativement aux taux d'expression de HOXC4 et TDRD1 dans un échantillon provenant d'un individu ne souffrant pas d'un cancer de la prostate ou comparativement à une valeur de référence indicative d'un taux d'expression non pathologique ; et dans lequel l'étape (c) comprend l'établissement de la présence ou de l'absence d'un cancer de la prostate (CaP) en se basant sur la régulation positive des taux d'expression de DLX1, HOXC6, HOXC4 et TDRD1 dans ledit échantillon.

2. Procédé selon la revendication 1, dans lequel l'établissement d'un cancer de la prostate (CaP) comprend l'établissement d'un cancer de la prostate de haut grade (score de Gleason ≥ 7) ou de bas grade (score de Gleason < 7).

3. Procédé selon l'une quelconque de la revendication 1 ou de la revendication 2, dans lequel l'établissement d'un cancer de la prostate (CaP) comprend l'établissement d'un pourcentage de carottes biopsiques positives ≥ 33 %.

4. Procédé selon la revendication 1, dans lequel l'établissement d'un cancer de la prostate (CaP) comprend l'établissement d'un stade clinique ≥ T2 selon les critères d'Epstein.

5. Procédé selon l'une quelconque des revendications 1 à 4, dans lequel l'établissement d'un cancer de la prostate (CaP) comprend l'établissement d'un cancer de la prostate de haut grade (score de Gleason ≥ 7), l'établissement d'un pourcentage de carottes biopsiques positives ≥ 33 % et l'établissement d'un stade clinique ≥ T2 selon les critères d'Epstein.

6. Procédé selon l'une quelconque des revendications 1 à 5, dans lequel la détermination des taux d'expression comprend la détermination des taux d'expression d'ARNm et/ou dans lequel la détermination des taux d'expression comprend la détermination des taux de protéine.

7. Procédé selon l'une quelconque des revendications 1 à 6, dans lequel ledit échantillon est un échantillon choisi dans le groupe constitué de l'urine, d'un dérivé de l'urine, d'un liquide prostatique, d'un dérivé de liquide prostatique, d'un éjaculat et d'un dérivé d'éjaculat.

8. Utilisation d'une combinaison d'un marqueur d'expression DLX1, d'un marqueur d'expression HOXC6, d'un marqueur d'expression HOXC4 et d'un marqueur d'expression TDRD1 pour l'établissement *in vitro* d'un cancer de la prostate (CaP).

9. Utilisation selon la revendication 8, dans laquelle l'établissement d'un cancer de la prostate comprend l'établissement d'un cancer de la prostate dans un échantillon choisi dans le groupe constitué de l'urine, d'un dérivé de l'urine, d'un liquide prostatique, d'un dérivé de liquide prostatique, d'un éjaculat et d'un dérivé d'éjaculat.

10. Utilisation d'un kit d'éléments dans un procédé selon l'une quelconque des revendications 1 à 7 pour l'établissement *in vitro* d'un cancer de la prostate (CaP) dans un échantillon provenant d'un individu humain suspecté de ou souffrant d'un cancer de la prostate, le kit comprenant :
- un moyen d'analyse des taux d'expression pour déterminer les taux d'expression de HOXC6 et DLX1 ;
- des instructions pour l'utilisation,
le kit comprenant en outre un moyen d'analyse des taux d'expression pour déterminer les taux d'expression de HOXC4 et TDRD1.


### SEQUENCE LISTING

<110> NovioGendix Research B.V.
<120> COMBINATIONS OF MOLECULAR MARKERS IN PROSTATE CANCER PROVIDING A DIAGNOSTIC TOOL WITH IMPROVED SENSITIVITY/SPECIFICITY
<130> 4/2QK29/12P
<150> PCT/EP2013/066889
   <151> 2013-08-13
<160> 14
<170> PatentIn version 3.5
<210> 1
   <211> 2300
   <212> DNA
   <213> Homo sapiens
<220>
   <221> misc_feature
   <223> cDNA sequence of the variant 1 of the HOXC4 gene (NM_014620.4, NP_055435.2)
<400> 1
   ttattgtggt ttgtccgttc cgagcgctcc gcagaacagt cctccctgta agagcctaac      60
   cattgccagg gaaacctgcc ctgggcgctc ccttcattag cagtattttt tttaaattaa     120
   tctgattaat aattattttt cccccattta attttttttc ctcccaggtg gagttgccga     180
   agctgggggc agctggggag ggtggggatg ggaggggaga gacagaagtt gagggcatct     240
   ctctcttcct tcccgaccct ctggccccca aggggcagga ggaatgcagg agcaggagtt     300
   gagcttggga gctgcagatg cctccgcccc tcctctctcc caggctcttc ctcctgcccc     360
   cttcttgcaa ctctccttaa ttttgtttgg cttttggatg attataatta tttttatttt     420
   tgaatttata taaagtatat gtgtgtgtgt gtggagctga gacaggctcg gcagcggcac     480
   agaatgaggg aagacgagaa agagagtggg agagagagag gcagagaggg agagagggag     540
   agtgacagca gcgctcgcgg gggctcaacc cccagacctc cagaaatgac gtcagaatca     600
   tttgcatccc gctgcctcta cctgcctggt ccagctggga ccctgcctcg ccggccgcat     660
   ggccagaggg ttggaaatta atgatcatga gctcgtattt gatggactct aactacatcg     720
   atccgaaatt tcctccatgc gaagaatatt cgcaaaatag ctacatccct gaacacagtc     780
   cggaatatta cggccggacc agggaatcgg gattccagca tcaccaccag gagctgtacc     840
   caccaccgcc tccgcgccct agctaccctg agcgccagta tagctgcacc agtctccagg     900
   ggcccggcaa ttcgcgaggc cacgggccgg cccaggcggg ccaccaccac cccgagaaat     960
   cacagtcgct ctgcgagccg gcgcctctct caggcgcctc cgcctccccg tccccagccc    1020
   cgccagcctg cagccagcca gcccccgacc atccctccag cgccgccagc aagcaaccca    1080
   tagtctaccc atggatgaaa aaaattcacg ttagcacggt gaaccccaat tataacggag    1140
   gggaacccaa gcgctcgagg acagcctata cccggcagca agtcctggaa ttagagaaag    1200
   agtttcatta caaccgctac ctgacccgaa ggagaaggat cgagatcgcc cactcgctgt    1260
   gcctctctga gaggcagatc aaaatctggt tccaaaaccg tcgcatgaaa tggaagaagg    1320
   accaccgact ccccaacacc aaagtcaggt cagcaccccc ggccggcgct gcgcccagca    1380
   ccctttcggc agctaccccg ggtacttctg aagaccactc ccagagcgcc acgccgccgg    1440
   agcagcaacg ggcagaggac attaccaggt tataaaacat aactcacacc cctgccccca    1500
   ccccatgccc ccaccctccc ctcacacaca aattgactct tatttataga atttaatata    1560
   tatatatata tatatatata taggttcttt tctctcttcc tctcaccttg tcccttgtca    1620
   gttccaaaca gacaaaacag ataaacaaac aagccccctg ccctcctctc cctcccactg    1680
   ttaaggaccc ttttaagcat gtgatgttgt cttagcatgg tacctgctgg gtgttttttt    1740
   ttaaaaggcc attttggggg gttatttatt ttttaagaaa aaaagctgca aaaattatat    1800
   attgcaaggt gtgatggtct ggcttgggtg aatttcaggg gaaatgagga aaagaaaaaa    1860
   ggaaagaaat tttaaagcca attctcatcc ttctcctcct cctccttccc cccctctttc    1920
   cttaggcctt ttgcattgaa aatgcaccag gggaggttag tgagggggaa gtcattttaa    1980
   ggagaacaaa gctatgaagt tcttttgtat tattgttggg ggggggtgtg ggaggagagg    2040
   gggcgaagac agcagacaaa gctaaatgca tctggagagc ctctcagagc tgttcagttt    2100
   gaggagccaa aagaaaatca aaatgaactt tcagttcaga gaggcagtct ataggtagaa    2160
   tctctcccca cccctatcgt ggttattgtg tttttggact gaatttactt gattattgta    2220
   aaacttgcaa taaagaattt tagtgtcgat gtgaaatgcc ccgtgatcaa taataaacca    2280
   gtggatgtga attagtttta                                                2300
<210> 2
   <211> 264
   <212> PRT
   <213> Homo sapiens
<220>
   <221> misc_feature
   <223> amino acid sequence of the variant 1 of the HOXC4 gene (NM_014620.4, NP_055435.2)
<400> 2
   Met Ile Met Ser Ser Tyr Leu Met Asp Ser Asn Tyr Ile Asp Pro Lys
   1               5                   10                  15
   Phe Pro Pro Cys Glu Glu Tyr Ser Gln Asn Ser Tyr Ile Pro Glu His
               20                  25                  30
   Ser Pro Glu Tyr Tyr Gly Arg Thr Arg Glu Ser Gly Phe Gln His His
           35                  40                  45
   His Gln Glu Leu Tyr Pro Pro Pro Pro Pro Arg Pro Ser Tyr Pro Glu
       50                  55                  60
   Arg Gln Tyr Ser Cys Thr Ser Leu Gln Gly Pro Gly Asn Ser Arg Gly
   65                  70                  75                  80
   His Gly Pro Ala Gln Ala Gly His His His Pro Glu Lys Ser Gln Ser
                   85                  90                  95
   Leu Cys Glu Pro Ala Pro Leu Ser Gly Ala Ser Ala Ser Pro Ser Pro
               100                 105                 110
   Ala Pro Pro Ala Cys Ser Gln Pro Ala Pro Asp His Pro Ser Ser Ala
           115                 120                 125
   Ala Ser Lys Gln Pro Ile Val Tyr Pro Trp Met Lys Lys Ile His Val
       130                 135                 140
   Ser Thr Val Asn Pro Asn Tyr Asn Gly Gly Glu Pro Lys Arg Ser Arg
   145                 150                 155                 160
   Thr Ala Tyr Thr Arg Gln Gln Val Leu Glu Leu Glu Lys Glu Phe His
                   165                 170                 175
   Tyr Asn Arg Tyr Leu Thr Arg Arg Arg Arg Ile Glu Ile Ala His Ser
               180                 185                 190
   Leu Cys Leu Ser Glu Arg Gln Ile Lys Ile Trp Phe Gln Asn Arg Arg
           195                 200                 205
   Met Lys Trp Lys Lys Asp His Arg Leu Pro Asn Thr Lys Val Arg Ser
       210                 215                 220
   Ala Pro Pro Ala Gly Ala Ala Pro Ser Thr Leu Ser Ala Ala Thr Pro
   225                 230                 235                 240
   Gly Thr Ser Glu Asp His Ser Gln Ser Ala Thr Pro Pro Glu Gln Gln
                   245                 250                 255
   Arg Ala Glu Asp Ile Thr Arg Leu
               260
<210> 3
   <211> 1689
   <212> DNA
   <213> Homo sapiens
<220>
   <221> misc_feature
   <223> cDNA sequence of the variant 2 of the HOXC4 gene (NM_153633.2, NP_705897.1)
<400> 3
   agaaaaacga caaagcgaga aaaattattt tccactccag aaattaatga tcatgagctc      60
   gtatttgatg gactctaact acatcgatcc gaaatttcct ccatgcgaag aatattcgca     120
   aaatagctac atccctgaac acagtccgga atattacggc cggaccaggg aatcgggatt     180
   ccagcatcac caccaggagc tgtacccacc accgcctccg cgccctagct accctgagcg     240
   ccagtatagc tgcaccagtc tccaggggcc cggcaattcg cgaggccacg ggccggccca     300
   ggcgggccac caccaccccg agaaatcaca gtcgctctgc gagccggcgc ctctctcagg     360
   cgcctccgcc tccccgtccc cagccccgcc agcctgcagc cagccagccc ccgaccatcc     420
   ctccagcgcc gccagcaagc aacccatagt ctacccatgg atgaaaaaaa ttcacgttag     480
   cacggtgaac cccaattata acggagggga acccaagcgc tcgaggacag cctatacccg     540
   gcagcaagtc ctggaattag agaaagagtt tcattacaac cgctacctga cccgaaggag     600
   aaggatcgag atcgcccact cgctgtgcct ctctgagagg cagatcaaaa tctggttcca     660
   aaaccgtcgc atgaaatgga agaaggacca ccgactcccc aacaccaaag tcaggtcagc     720
   acccccggcc ggcgctgcgc ccagcaccct ttcggcagct accccgggta cttctgaaga     780
   ccactcccag agcgccacgc cgccggagca gcaacgggca gaggacatta ccaggttata     840
   aaacataact cacacccctg cccccacccc atgcccccac cctcccctca cacacaaatt     900
   gactcttatt tatagaattt aatatatata tatatatata tatatatagg ttcttttctc     960
   tcttcctctc accttgtccc ttgtcagttc caaacagaca aaacagataa acaaacaagc    1020
   cccctgccct cctctccctc ccactgttaa ggaccctttt aagcatgtga tgttgtctta    1080
   gcatggtacc tgctgggtgt ttttttttaa aaggccattt tggggggtta tttatttttt   1140
   aagaaaaaaa gctgcaaaaa ttatatattg caaggtgtga tggtctggct tgggtgaatt    1200
   tcaggggaaa tgaggaaaag aaaaaaggaa agaaatttta aagccaattc tcatccttct    1260
   cctcctcctc cttccccccc tctttcctta ggccttttgc attgaaaatg caccagggga    1320
   ggttagtgag ggggaagtca ttttaaggag aacaaagcta tgaagttctt ttgtattatt    1380
   gttggggggg ggtgtgggag gagagggggc gaagacagca gacaaagcta aatgcatctg    1440
   gagagcctct cagagctgtt cagtttgagg agccaaaaga aaatcaaaat gaactttcag    1500
   ttcagagagg cagtctatag gtagaatctc tccccacccc tatcgtggtt attgtgtttt    1560
   tggactgaat ttacttgatt attgtaaaac ttgcaataaa gaattttagt gtcgatgtga    1620
   aatgccccgt gatcaataat aaaccagtgg atgtgaatta gttttaaaaa aaaaaaaaaa    1680
   aaaaaaaaa                                                            1689
<210> 4
   <211> 264
   <212> PRT
   <213> Homo sapiens
<220>
   <221> misc_feature
   <223> amino acid sequence of the variant 2 of the HOXC4 gene (NM_153633.2, NP_705897.1)
<400> 4
   Met Ile Met Ser Ser Tyr Leu Met Asp Ser Asn Tyr Ile Asp Pro Lys
   1               5                   10                  15
   Phe Pro Pro Cys Glu Glu Tyr Ser Gln Asn Ser Tyr Ile Pro Glu His
               20                  25                  30
   Ser Pro Glu Tyr Tyr Gly Arg Thr Arg Glu Ser Gly Phe Gln His His
           35                  40                  45
   His Gln Glu Leu Tyr Pro Pro Pro Pro Pro Arg Pro Ser Tyr Pro Glu
       50                  55                  60
   Arg Gln Tyr Ser Cys Thr Ser Leu Gln Gly Pro Gly Asn Ser Arg Gly
   65                  70                  75                  80
   His Gly Pro Ala Gln Ala Gly His His His Pro Glu Lys Ser Gln Ser
                   85                  90                  95
   Leu Cys Glu Pro Ala Pro Leu Ser Gly Ala Ser Ala Ser Pro Ser Pro
               100                 105                 110
   Ala Pro Pro Ala Cys Ser Gln Pro Ala Pro Asp His Pro Ser Ser Ala
           115                 120                 125
   Ala Ser Lys Gln Pro Ile Val Tyr Pro Trp Met Lys Lys Ile His Val
       130                 135                 140
   Ser Thr Val Asn Pro Asn Tyr Asn Gly Gly Glu Pro Lys Arg Ser Arg
   145                 150                 155                 160
   Thr Ala Tyr Thr Arg Gln Gln Val Leu Glu Leu Glu Lys Glu Phe His
                   165                 170                 175
   Tyr Asn Arg Tyr Leu Thr Arg Arg Arg Arg Ile Glu Ile Ala His Ser
               180                 185                 190
   Leu Cys Leu Ser Glu Arg Gln Ile Lys Ile Trp Phe Gln Asn Arg Arg
           195                 200                 205
   Met Lys Trp Lys Lys Asp His Arg Leu Pro Asn Thr Lys Val Arg Ser
       210                 215                 220
   Ala Pro Pro Ala Gly Ala Ala Pro Ser Thr Leu Ser Ala Ala Thr Pro
   225                 230                 235                 240
   Gly Thr Ser Glu Asp His Ser Gln Ser Ala Thr Pro Pro Glu Gln Gln
                   245                 250                 255
   Arg Ala Glu Asp Ile Thr Arg Leu
               260
<210> 5
   <211> 1681
   <212> DNA
   <213> Homo sapiens
<220>
   <221> misc_feature
   <223> cDNA sequence of the variant 1 of the HOXC6 gene (NM_004503.3, NP_004494.1)
<400> 5
   ttttgtctgt cctggattgg agccgtccct ataaccatct agttccgagt acaaactgga      60
   gacagaaata aatattaaag aaatcataga ccgaccaggt aaaggcaaag ggatgaattc     120
   ctacttcact aacccttcct tatcctgcca cctcgccggg ggccaggacg tcctccccaa     180
   cgtcgccctc aattccaccg cctatgatcc agtgaggcat ttctcgacct atggagcggc     240
   cgttgcccag aaccggatct actcgactcc cttttattcg ccacaggaga atgtcgtgtt     300
   cagttccagc cgggggccgt atgactatgg atctaattcc ttttaccagg agaaagacat     360
   gctctcaaac tgcagacaaa acaccttagg acataacaca cagacctcaa tcgctcagga     420
   ttttagttct gagcagggca ggactgcgcc ccaggaccag aaagccagta tccagattta     480
   cccctggatg cagcgaatga attcgcacag tggggtcggc tacggagcgg accggaggcg     540
   cggccgccag atctactcgc ggtaccagac cctggaactg gagaaggaat ttcacttcaa     600
   tcgctaccta acgcggcgcc ggcgcatcga gatcgccaac gcgctttgcc tgaccgagcg     660
   acagatcaaa atctggttcc agaaccgccg gatgaagtgg aaaaaagaat ctaatctcac     720
   atccactctc tcggggggcg gcggaggggc caccgccgac agcctgggcg gaaaagagga     780
   aaagcgggaa gagacagaag aggagaagca gaaagagtga ccaggactgt ccctgccacc     840
   cctctctccc tttctccctc gctccccacc aactctcccc taatcacaca ctctgtattt     900
   atcactggca caattgatgt gttttgattc cctaaaacaa aattagggag tcaaacgtgg     960
   acctgaaagt cagctctgga ccccctccct caccgcacaa ctctctttca ccacgcgcct    1020
   cctcctcctc gctcccttgc tagctcgttc tcggcttgtc tacaggccct tttccccgtc    1080
   caggccttgg gggctcggac cctgaactca gactctacag attgccctcc aagtgaggac    1140
   ttggctcccc cactccttcg acgcccccac ccccgccccc cgtgcagaga gccggctcct    1200
   gggcctgctg gggcctctgc tccagggcct cagggcccgg cctggcagcc ggggagggcc    1260
   ggaggcccaa ggagggcgcg ccttggcccc acaccaaccc ccagggcctc cccgcagtcc    1320
   ctgcctagcc cctctgcccc agcaaatgcc cagcccaggc aaattgtatt taaagaatcc    1380
   tgggggtcat tatggcattt tacaaactgt gaccgtttct gtgtgaagat ttttagctgt    1440
   atttgtggtc tctgtattta tatttatgtt tagcaccgtc agtgttccta tccaatttca    1500
   aaaaaggaaa aaaaagaggg aaaattacaa aaagagagaa aaaaagtgaa tgacgtttgt    1560
   ttagccagta ggagaaaata aataaataaa taaatccctt cgtgttaccc tcctgtataa    1620
   atccaacctc tgggtccgtt ctcgaatatt taataaaact gatattattt ttaaaacttt    1680
   a                                                                    1681
<210> 6
   <211> 235
   <212> PRT
   <213> Homo sapiens
<220>
   <221> misc_feature
   <223> amino acid sequence of the variant 1 of the HOXC6 gene (NM_004503.3, NP_004494.1)
<400> 6
   Met Asn Ser Tyr Phe Thr Asn Pro Ser Leu Ser Cys His Leu Ala Gly
   1               5                   10                  15
   Gly Gln Asp Val Leu Pro Asn Val Ala Leu Asn Ser Thr Ala Tyr Asp
               20                  25                  30
   Pro Val Arg His Phe Ser Thr Tyr Gly Ala Ala Val Ala Gln Asn Arg
           35                  40                  45
   Ile Tyr Ser Thr Pro Phe Tyr Ser Pro Gln Glu Asn Val Val Phe Ser
       50                  55                  60
   Ser Ser Arg Gly Pro Tyr Asp Tyr Gly Ser Asn Ser Phe Tyr Gln Glu
   65                  70                  75                  80
   Lys Asp Met Leu Ser Asn Cys Arg Gln Asn Thr Leu Gly His Asn Thr
                   85                  90                  95
   Gln Thr Ser Ile Ala Gln Asp Phe Ser Ser Glu Gln Gly Arg Thr Ala
               100                 105                 110
   Pro Gln Asp Gln Lys Ala Ser Ile Gln Ile Tyr Pro Trp Met Gln Arg
               115                 120                 125
   Met Asn Ser His Ser Gly Val Gly Tyr Gly Ala Asp Arg Arg Arg Gly
           130                 135                 140
   Arg Gln Ile Tyr Ser Arg Tyr Gln Thr Leu Glu Leu Glu Lys Glu Phe
   145                 150                 155                 160
   His Phe Asn Arg Tyr Leu Thr Arg Arg Arg Arg Ile Glu Ile Ala Asn
                   165                 170                 175
   Ala Leu Cys Leu Thr Glu Arg Gln Ile Lys Ile Trp Phe Gln Asn Arg
               180                 185                 190
   Arg Met Lys Trp Lys Lys Glu Ser Asn Leu Thr Ser Thr Leu Ser Gly
           195                 200                 205
   Gly Gly Gly Gly Ala Thr Ala Asp Ser Leu Gly Gly Lys Glu Glu Lys
       210                 215                 220
   Arg Glu Glu Thr Glu Glu Glu Lys Gln Lys Glu
   225                 230                 235
<210> 7
   <211> 2072
   <212> DNA
   <213> Homo sapiens
<220>
   <221> misc_feature
   <223> cDNA sequence of the variant 2 of the HOXC6 gene (NM_153693.3, NP_710160.1)
<400> 7
   ttattgtggt ttgtccgttc cgagcgctcc gcagaacagt cctccctgta agagcctaac     60
   cattgccagg gaaacctgcc ctgggcgctc ccttcattag cagtattttt tttaaattaa    120
   tctgattaat aattattttt cccccattta attttttttc ctcccaggtg gagttgccga    180
   agctgggggc agctggggag ggtggggatg ggaggggaga gacagaagtt gagggcatct    240
   ctctcttcct tcccgaccct ctggccccca aggggcagga ggaatgcagg agcaggagtt    300
   gagcttggga gctgcagatg cctccgcccc tcctctctcc caggctcttc ctcctgcccc    360
   cttcttgcaa ctctccttaa ttttgtttgg cttttggatg attataatta tttttatttt    420
   tgaatttata taaagtatat gtgtgtgtgt gtggagctga gacaggctcg gcagcggcac    480
   agaatgaggg aagacgagaa agagagtggg agagagagag gcagagaggg agagagggag    540
   agtgacagca gcgctcggac gtcctcccca acgtcgccct caattccacc gcctatgatc    600
   cagtgaggca tttctcgacc tatggagcgg ccgttgccca gaaccggatc tactcgactc    660
   ccttttattc gccacaggag aatgtcgtgt tcagttccag ccgggggccg tatgactatg    720
   gatctaattc cttttaccag gagaaagaca tgctctcaaa ctgcagacaa aacaccttag    780
   gacataacac acagacctca atcgctcagg attttagttc tgagcagggc aggactgcgc    840
   cccaggacca gaaagccagt atccagattt acccctggat gcagcgaatg aattcgcaca    900
   gtggggtcgg ctacggagcg gaccggaggc gcggccgcca gatctactcg cggtaccaga    960
   ccctggaact ggagaaggaa tttcacttca atcgctacct aacgcggcgc cggcgcatcg   1020
   agatcgccaa cgcgctttgc ctgaccgagc gacagatcaa aatctggttc cagaaccgcc   1080
   ggatgaagtg gaaaaaagaa tctaatctca catccactct ctcggggggc ggcggagggg   1140
   ccaccgccga cagcctgggc ggaaaagagg aaaagcggga agagacagaa gaggagaagc   1200
   agaaagagtg accaggactg tccctgccac ccctctctcc ctttctccct cgctccccac   1260
   caactctccc ctaatcacac actctgtatt tatcactggc acaattgatg tgttttgatt   1320
   ccctaaaaca aaattaggga gtcaaacgtg gacctgaaag tcagctctgg accccctccc   1380
   tcaccgcaca actctctttc accacgcgcc tcctcctcct cgctcccttg ctagctcgtt   1440
   ctcggcttgt ctacaggccc ttttccccgt ccaggccttg ggggctcgga ccctgaactc   1500
   agactctaca gattgccctc caagtgagga cttggctccc ccactccttc gacgccccca   1560
   cccccgcccc ccgtgcagag agccggctcc tgggcctgct ggggcctctg ctccagggcc     1620
   tcagggcccg gcctggcagc cggggagggc cggaggccca aggagggcgc gccttggccc     1680
   cacaccaacc cccagggcct ccccgcagtc cctgcctagc ccctctgccc cagcaaatgc     1740
   ccagcccagg caaattgtat ttaaagaatc ctgggggtca ttatggcatt ttacaaactg     1800
   tgaccgtttc tgtgtgaaga tttttagctg tatttgtggt ctctgtattt atatttatgt     1860
   ttagcaccgt cagtgttcct atccaatttc aaaaaaggaa aaaaaagagg gaaaattaca     1920
   aaaagagaga aaaaaagtga atgacgtttg tttagccagt aggagaaaat aaataaataa     1980
   ataaatccct tcgtgttacc ctcctgtata aatccaacct ctgggtccgt tctcgaatat     2040
   ttaataaaac tgatattatt tttaaaactt ta                                    2072
<210> 8
   <211> 153
   <212> PRT
   <213> Homo sapiens
<220>
   <221> misc_feature
   <223> mino acid sequence of the variant 2 of the HOXC6 gene (NM_153693.3, NP_710160.1)
<400> 8
   Met Leu Ser Asn Cys Arg Gln Asn Thr Leu Gly His Asn Thr Gln Thr
   1               5                   10                  15
   Ser Ile Ala Gln Asp Phe Ser Ser Glu Gln Gly Arg Thr Ala Pro Gln
               20                  25                  30
   Asp Gln Lys Ala Ser Ile Gln Ile Tyr Pro Trp Met Gln Arg Met Asn
           35                  40                  45
   Ser His Ser Gly Val Gly Tyr Gly Ala Asp Arg Arg Arg Gly Arg Gln
       50                  55                  60
   Ile Tyr Ser Arg Tyr Gln Thr Leu Glu Leu Glu Lys Glu Phe His Phe
   65                  70                  75                  80
   Asn Arg Tyr Leu Thr Arg Arg Arg Arg Ile Glu Ile Ala Asn Ala Leu
                   85                  90                  95
   Cys Leu Thr Glu Arg Gln Ile Lys Ile Trp Phe Gln Asn Arg Arg Met
               100                 105                 110
   Lys Trp Lys Lys Glu Ser Asn Leu Thr Ser Thr Leu Ser Gly Gly Gly
           115                 120                 125
   Gly Gly Ala Thr Ala Asp Ser Leu Gly Gly Lys Glu Glu Lys Arg Glu
       130                 135                 140
   Glu Thr Glu Glu Glu Lys Gln Lys Glu
   145                 150
<210> 9
   <211> 2403
   <212> DNA
   <213> Homo sapiens
<220>
   <221> misc_feature
   <223> cDNA sequence of transcript variant one of the DLX1 gene (NM_178120, NP_835221)
<400> 9
   aagctttgaa ccgagtttgg ggagctcagc agcatcatgc ttagactttt caaagagaca       60
   aactccattt tcttatgaat ggaaagtgaa aacccctgtt ccgcttaaat tgggttcctt      120
   cctgtcctga gaaacataga gacccccaaa agggaagcag aggagagaaa gtcccacacc 180
   cagaccccgc gagaagagat gaccatgacc accatgccag aaagtctcaa cagccccgtg 240
   tcgggcaagg cggtgtttat ggagtttggg ccgcccaacc agcaaatgtc tccttctccc 300
   atgtcccacg ggcactactc catgcactgt ttacactcgg cgggccattc gcagcccgac 360
   ggcgcctaca gctcagcctc gtccttctcc cgaccgctgg gctaccccta cgtcaactcg 420
   gtcagcagcc acgcatccag cccctacatc agttcggtgc agtcctaccc gggcagcgcc 480
   agcctcgccc agagccgcct ggaggaccca ggggcggact cggagaagag cacggtggtg 540
   gaaggcggtg aagtgcgctt caatggcaag ggaaaaaaga tccgtaaacc caggacgatt 600
   tattccagtt tgcagttgca ggctttgaac cggaggttcc agcaaactca gtacctagct 660
   ctgccggaga gggcggagct cgcggcctct ttgggactca cacagactca ggtcaagatc 720
   tggttccaaa acaagcgatc caagttcaag aagctgatga agcagggtgg ggcggctctg 780
   gagggtagtg cgttggccaa cggtcgggcc ctgtctgctg gctccccacc cgtgccgccc 840
   ggctggaacc ctaactcttc atccgggaag ggctcaggag gaaacgcggg ctcctatatc 900
   cccagctaca catcgtggta cccttcagcg caccaagaag ctatgcagca accccaactt 960
   atgtgaggtt gcccgcccgt ctccttcttg tctccccggc ccaggtccct cccgcctcca 1020
   ggtccatcca tcccgtccgg aaaagaagga cccagaggga agaaggaaca gtggaggcgg 1080
   gacgccctcc atctcctcgg agccccgcga ggtccggccc agcaacttcc cggcatccgc 1140
   gctctagcct gaaccctggc ctgggccgag cagtggcagc agagagtggc ctcggaggga 1200
   agccactgcc acctgagaca gcccaagcag caagataaac ccgctccacc cgacccgccg 1260
   accttcagct ttgtgggact atcaggaaaa aacaaaacaa aaacaaaatg tagaaaaagc 1320
   aaaagctctt ttctgtcctg tcagtctcct gtctcctttt gctctgtctg tgcgctggta 1380
   aagtccaggt cctcatccgt ccgctgtcct cattctgcgg cctcagcaaa aagccacaag 1440
   gtctgagcgg cccgggtcct gccgggctga ccatctccgg atcctgggac actctgcctg 1500
   accatctgtg tagctggtgt gggaatctgg gggcattgga gggagggggt tttatttatt 1560
   gagaaatgga cttcgcctga ggctgtttgc caattcaggg ttctgctggg cgcaaggaac 1620
   gcactgttca aacgcactgt ttactttaag cgcacgggga gaaacgaata aggaggacgt 1680
   ggtgattttt aatttataca gtaacttttg tacttctctg gtatggagag tttggagccg 1740
   aatgatttgc attttttaca tgtccgacat tatttaataa ataattttta aaagaaaaga 1800
   acgataaatg aagccaacat gattttctca tttcgggagg aactctgttg cttcgcctgg 1860
   acaagaagga aaatgctgat ttcctccttg ggtagaaaga gggagcgagg gcaaatgggg 1920
   agtagagaga aaacaggcga gaacaagcac tctaattcca gtgggcttta aaataagaca 1980
   aaatcagctt tacaacaatc cctagaggct cgaccacaga ataatgccag tcaccaccct 2040
   gaacgcacaa tctccagtgc aggatctaat gactgtacat attattgtta ttattattat 2100
   tgttattatt gttgttctgt aaacatgttg cacaagctta gcctttttgc gttctgttgt 2160
   gtgtggctgt aaaaccccat gctttgtgaa atgagaatct tgacattttt cttgtgaaat 2220
   ttggaaaatg tgatcaattg aaatcaactg tgttttgtgt tctctatgtc aaagtttagt 2280
   tttatattga gaatgttaac ttattgcttt gtatcttggg aaaaaaactt tgtaaataag 2340
   ttataaagtt tctttgagac agtaaaatta tgatttcttg aaaaaaaaaa aaaaaaaaaa 2400
   aaa 2403
<210> 10
   <211> 255
   <212> PRT
   <213> Homo sapiens
<220>
   <221> misc_feature
   <223> amino acid sequence of transcript variant one of the DLX1 gene (NM_178120, NP_835221)
<400> 10
   Met Thr Met Thr Thr Met Pro Glu Ser Leu Asn Ser Pro Val Ser Gly
   1 5 10 15
   Lys Ala Val Phe Met Glu Phe Gly Pro Pro Asn Gln Gln Met Ser Pro
                   20                  25                  30
   Ser Pro Met Ser His Gly His Tyr Ser Met His Cys Leu His Ser Ala
               35                  40                  45
   Gly His Ser Gln Pro Asp Gly Ala Tyr Ser Ser Ala Ser Ser Phe Ser
       50                  55                  60
   Arg Pro Leu Gly Tyr Pro Tyr Val Asn Ser Val Ser Ser His Ala Ser
   65                  70                  75                  80
   Ser Pro Tyr Ile Ser Ser Val Gln Ser Tyr Pro Gly Ser Ala Ser Leu
                   85                  90                  95
   Ala Gln Ser Arg Leu Glu Asp Pro Gly Ala Asp Ser Glu Lys Ser Thr
               100                 105                 110
   Val Val Glu Gly Gly Glu Val Arg Phe Asn Gly Lys Gly Lys Lys Ile
           115                 120                 125
   Arg Lys Pro Arg Thr Ile Tyr Ser Ser Leu Gln Leu Gln Ala Leu Asn
       130                 135                 140
   Arg Arg Phe Gln Gln Thr Gln Tyr Leu Ala Leu Pro Glu Arg Ala Glu
   145                 150                 155                 160
   Leu Ala Ala Ser Leu Gly Leu Thr Gln Thr Gln Val Lys Ile Trp Phe
                   165                 170                 175
   Gln Asn Lys Arg Ser Lys Phe Lys Lys Leu Met Lys Gln Gly Gly Ala
               180                 185                 190
   Ala Leu Glu Gly Ser Ala Leu Ala Asn Gly Arg Ala Leu Ser Ala Gly
           195                 200                 205
   Ser Pro Pro Val Pro Pro Gly Trp Asn Pro Asn Ser Ser Ser Gly Lys
       210                 215                 220
   Gly Ser Gly Gly Asn Ala Gly Ser Tyr Ile Pro Ser Tyr Thr Ser Trp
   225                 230                 235                 240
   Tyr Pro Ser Ala His Gln Glu Ala Met Gln Gln Pro Gln Leu Met
                   245                 250                 255
<210> 11
   <211> 2203
   <212> DNA
   <213> Homo sapiens
<220>
   <221> misc_feature
   <223> cDNA sequence of transcript variant two of the DLX1 gene (NM_001038493.1, NP_001033582.1)
<400> 11
   aagctttgaa ccgagtttgg ggagctcagc agcatcatgc ttagactttt caaagagaca      60
   aactccattt tcttatgaat ggaaagtgaa aacccctgtt ccgcttaaat tgggttcctt     120
   cctgtcctga gaaacataga gacccccaaa agggaagcag aggagagaaa gtcccacacc     180
   cagaccccgc gagaagagat gaccatgacc accatgccag aaagtctcaa cagccccgtg     240
   tcgggcaagg cggtgtttat ggagtttggg ccgcccaacc agcaaatgtc tccttctccc     300
   atgtcccacg ggcactactc catgcactgt ttacactcgg cgggccattc gcagcccgac     360
   ggcgcctaca gctcagcctc gtccttctcc cgaccgctgg gctaccccta cgtcaactcg     420
   gtcagcagcc acgcatccag cccctacatc agttcggtgc agtcctaccc gggcagcgcc     480
   agcctcgccc agagccgcct ggaggaccca ggtcaagatc tggttccaaa acaagcgatc     540
   caagttcaag aagctgatga agcagggtgg ggcggctctg gagggtagtg cgttggccaa     600
   cggtcgggcc ctgtctgctg gctccccacc cgtgccgccc ggctggaacc ctaactcttc     660
   atccgggaag ggctcaggag gaaacgcggg ctcctatatc cccagctaca catcgtggta      720
   cccttcagcg caccaagaag ctatgcagca accccaactt atgtgaggtt gcccgcccgt      780
   ctccttcttg tctccccggc ccaggtccct cccgcctcca ggtccatcca tcccgtccgg      840
   aaaagaagga cccagaggga agaaggaaca gtggaggcgg gacgccctcc atctcctcgg      900
   agccccgcga ggtccggccc agcaacttcc cggcatccgc gctctagcct gaaccctggc      960
   ctgggccgag cagtggcagc agagagtggc ctcggaggga agccactgcc acctgagaca     1020
   gcccaagcag caagataaac ccgctccacc cgacccgccg accttcagct ttgtgggact     1080
   atcaggaaaa aacaaaacaa aaacaaaatg tagaaaaagc aaaagctctt ttctgtcctg     1140
   tcagtctcct gtctcctttt gctctgtctg tgcgctggta aagtccaggt cctcatccgt     1200
   ccgctgtcct cattctgcgg cctcagcaaa aagccacaag gtctgagcgg cccgggtcct     1260
   gccgggctga ccatctccgg atcctgggac actctgcctg accatctgtg tagctggtgt     1320
   gggaatctgg gggcattgga gggagggggt tttatttatt gagaaatgga cttcgcctga     1380
   ggctgtttgc caattcaggg ttctgctggg cgcaaggaac gcactgttca aacgcactgt     1440
   ttactttaag cgcacgggga gaaacgaata aggaggacgt ggtgattttt aatttataca     1500
   gtaacttttg tacttctctg gtatggagag tttggagccg aatgatttgc attttttaca     1560
   tgtccgacat tatttaataa ataattttta aaagaaaaga acgataaatg aagccaacat     1620
   gattttctca tttcgggagg aactctgttg cttcgcctgg acaagaagga aaatgctgat     1680
   ttcctccttg ggtagaaaga gggagcgagg gcaaatgggg agtagagaga aaacaggcga     1740
   gaacaagcac tctaattcca gtgggcttta aaataagaca aaatcagctt tacaacaatc     1800
   cctagaggct cgaccacaga ataatgccag tcaccaccct gaacgcacaa tctccagtgc     1860
   aggatctaat gactgtacat attattgtta ttattattat tgttattatt gttgttctgt     1920
   aaacatgttg cacaagctta gcctttttgc gttctgttgt gtgtggctgt aaaaccccat     1980
   gctttgtgaa atgagaatct tgacattttt cttgtgaaat ttggaaaatg tgatcaattg     2040
   aaatcaactg tgttttgtgt tctctatgtc aaagtttagt tttatattga gaatgttaac     2100
   ttattgcttt gtatcttggg aaaaaaactt tgtaaataag ttataaagtt tctttgagac     2160
   agtaaaatta tgatttcttg aaaaaaaaaa aaaaaaaaaa aaa                      2203
<210> 12
   <211> 129
   <212> PRT
   <213> Homo sapiens
<220>
   <221> misc_feature
   <223> amino acid sequence of transcript variant two of the DLX1 gene (NM_001038493.1, NP_001033582.1)
<400> 12
   Met Thr Met Thr Thr Met Pro Glu Ser Leu Asn Ser Pro Val Ser Gly
   1               5                   10                  15
   Lys Ala Val Phe Met Glu Phe Gly Pro Pro Asn Gln Gln Met Ser Pro
               20                  25                  30
   Ser Pro Met Ser His Gly His Tyr Ser Met His Cys Leu His Ser Ala
           35                  40                  45
   Gly His Ser Gln Pro Asp Gly Ala Tyr Ser Ser Ala Ser Ser Phe Ser
       50                  55                  60
   Arg Pro Leu Gly Tyr Pro Tyr Val Asn Ser Val Ser Ser His Ala Ser
   65                  70                  75                  80
   Ser Pro Tyr Ile Ser Ser Val Gln Ser Tyr Pro Gly Ser Ala Ser Leu
                   85                  90                  95
   Ala Gln Ser Arg Leu Glu Asp Pro Gly Gln Asp Leu Val Pro Lys Gln
               100                 105                 110
   Ala Ile Gln Val Gln Glu Ala Asp Glu Ala Gly Trp Gly Gly Ser Gly
   115 120 125
   Gly
<210> 13
   <211> 4510
   <212> DNA
   <213> Homo sapiens
<220>
   <221> misc_feature
   <223> cDNA sequence of the TDRD1 gene (NM_198795.1, NP_942090.1)
<400> 13
   gctgaggcca ggagggcgca ctggggattg gaggcgaggg aagtgcaggg cgcatcccag      60
   gcggcagggc tcccagcatc ggcagtcgcc atcaccgcca gaccgcagag acaggttcgg     120
   atccgcggtc ctcttgcctc tttccaggcc tcgatgagtg ttaaatcgcc atttaatgtg     180
   atgtcaagaa ataatttgga agcacctcct tgtaagatga cagagccatt taattttgag     240
   aaaaatgaaa acaagcttcc accacatgag tctttaagaa gtcctggaac acttcctaac     300
   caccctaatt tcaggctgaa aagctcagag aatggaaata aaaagaacaa ttttttgctt     360
   tgtgagcaaa ccaaacaata tttggctagt caggaagaca attcagtttc ttcaaacccg     420
   aatggcatca acggagaagt agttggctcc aaaggagaca ggaaaaaatt gccagcagga     480
   aactcagtgt caccaccaag tgctgaaagt aattcaccac ccaaagaagt gaatattaag     540
   cctggaaata atgtacgtcc tgcaaaatca aaaaaactaa acaagttggt cgagaattcc     600
   ttgtccataa gtaatccagg gctcttcacc tccttaggac ctcctcttcg gtccacaact     660
   tgccatcgct gtggcctatt tggatcgctg aggtgctctc agtgcaagca gacctactat     720
   tgctccacag catgtcaaag aagagactgg tctgcacaca gcatcgtgtg caggcctgtt     780
   cagccaaatt tccacaaact tgaaaataaa tcatctattg aaacaaagga tgtggaggta     840
   aacaataaga gtgactgtcc acttggagtt actaaggaaa tagccatttg ggctgagaga     900
   ataatgtttt ctgatttgag aagtctacaa ctcaagaaaa ccatggaaat aaagggtacg     960
   gttaccgaat tcaaacaccc aggggacttc tacgtgcagt tatattcttc agaagtttta    1020
   gaatacatga accaactctc tgccagctta aaagaaacat atgcaaatgt gcatgaaaaa    1080
   gactatattc ctgttaaggg ggaagtttgt attgccaagt acactgttga tcagacctgg    1140
   aacagagcaa tcatacaaaa cgttgatgtg cagcaaaaga aggcacatgt cttatatatt    1200
   gattatggaa atgaagaaat aattccatta aacagaattt accacctcaa caggaacatt    1260
   gacttgtttc ctccttgtgc cataaagtgc tttgtagcca atgttatccc agcagaaggg    1320
   aattggagca gtgattgtat caaagctact aaaccactgt taatggagca gtactgctcc    1380
   ataaagattg tcgacatctt ggaagaggaa gtggttacct ttgctgtaga agttgagctg    1440
   ccaaattcag gaaaactttt agaccatgtg cttatagaaa tgggatatgg cttgaaaccc    1500
   agtggacaag attctaagaa ggaaaatgca gatcaaagtg atcctgaaga tgttggaaaa    1560
   atgacaactg aaaacaacat tgtcgtagac aaaagtgacc taatcccaaa agtgttaact    1620
   ttgaatgtag gtgatgagtt ttgtggtgtg gttgcccaca ttcaaacacc agaagacttc    1680
   ttttgtcaac aactgcaaag tggccgaaag cttgctgaac ttcaggcatc ccttagcaag    1740
   tactgtgatc agttgcctcc acgctctgat ttttatccag ccattggtga tatatgttgt    1800
   gctcagttct cagaggatga tcagtggtac cgtgcctctg ttttggctta cgcttctgaa    1860
   gaatctgtac tggtcggata tgtagattat ggaaactttg aaatccttag tttgatgaga    1920
   ctttgtccca taatcccaaa gttgttggaa ttgccaatgc aagctataaa gtgtgtacta    1980
   gcaggagtaa agccatcatt aggaatttgg actccagaag ctatttgtct catgaaaaaa    2040
   cttgtacaga acaaaataat cacagtgaaa gtggtggaca agttggaaaa cagttccctg    2100
   gtggagctta ttgataaatc cgagacgcct catgtcagtg ttagcaaagt tctcctagat    2160
   gcaggctttg ctgtgggaga acagagtatg gtgacagata aacccagtga cgtgaaagaa    2220
   accagtgttc ccttgggtgt ggaaggaaaa gtaaatccat tggagtggac atgggttgaa    2280
   cttggtgttg accaaacagt agatgttgtg gtctgtgtga tatatagtcc tggagaattt    2340
   tattgccatg tgcttaaaga ggatgcttta aagaaactca atgatttgaa caagtcatta     2400
   gcagaacact gccagcagaa gttacctaat ggtttcaagg cagagatagg acaaccttgt     2460
   tgtgcttttt ttgcaggtga tggtagttgg tatcgtgctt tagtcaagga aatcttacca     2520
   aatggacatg ttaaagtaca ttttgtggat tatggaaaca tcgaagaagt tactgcagat     2580
   gaactccgaa tgatatcatc aacattttta aaccttccct ttcagggaat acggtgccag     2640
   ttagcagata tacagtctag aaacaaacat tggtctgaag aagccataac aagattccag     2700
   atgtgtgttg ctgggataaa attgcaagcc agagtggttg aagtcactga aaatgggata     2760
   ggagttgaac tcaccgatct ctccacttgt tatcccagaa taattagtga tgttctgatt     2820
   gatgaacatc tggttttaaa atctgcttca ccacataaag acttaccaaa tgacagactt     2880
   gttaataaac atgagcttca agttcatgta cagggacttc aagctacctc ttcagctgag     2940
   caatggaaga cgatagaatt gccagtggat aaaactatac aagcaaatgt attagaaatc     3000
   ataagcccaa acttgtttta tgctctacca aaagggatgc cagaaaatca ggaaaagctg     3060
   tgcatgttga cagctgaatt attagaatac tgcaatgctc cgaaaagtcg accaccctat     3120
   agaccaagaa ttggagacgc atgctgtgcc aaatacacaa gtgatgattt ttggtatcgt     3180
   gcagttgttc tggggacatc agacactgat gtggaagtgc tctatgcaga ctatggaaac     3240
   attgaaaccc tgcctctttg cagagtgcaa ccaatcacct ctagccacct ggcgcttcct     3300
   ttccaaatta ttagatgttc acttgaagga ttaatggaat tgaatggaag ctcttctcaa     3360
   ttaataataa tgctattaaa aaatttcatg ttgaatcaga atgtaatgct ttctgtgaaa     3420
   ggaattacaa agaatgtcca tacagtgtca gttgagaaat gttctgagaa tgggactgtc     3480
   gatgtagctg ataagctagt gacatttggt ctggcaaaaa acatcacacc tcaaaggcag     3540
   agtgctttaa atacagaaaa gatgtatagg atgaattgct gctgcacaga gttacagaaa     3600
   caagttgaaa aacatgaaca tattcttctc ttcctcttaa acaattcaac caatcaaaat     3660
   aaatttattg aaatgaaaaa actgttaaaa aaaacagcat ctcttggagg taaaccctta     3720
   tgagacagga aacagcaaag gctagcttta ggagagaaag tacagcacct ggtgttttta     3780
   tttatgagaa ccttttcttt gtccactttc tctgtaatga ccttctatcc ctccgttttt     3840
   gcctgcctgc cattctccta ttaggttggt ggtttttatt ttcctctaag ttccttccac     3900
   caaataaata ttacgtaaaa aattcatacc aaatcaatga gaatactggc aaggaataca     3960
   tagggacttt ctgctatata tgtaactttt tattacttaa aggtaccgaa ggaaggccag     4020
   gtgcagtggc tcacgcccag cactttggga ggctgaggtg ggaggatccc ttgaggccag     4080
   gagttcaagg ttacagtgag ctatgatagt gccactgcac tccagcctgg gtgacagatt     4140
   ttgtcttaaa aaaaaaaaaa aaaaagttga tatgagtttt attttctgtc cgtttgaaat     4200
   attttgtaat attccctgca ttctctgtcg tctgcctctt ccacataatg tcctttgctt     4260
   tcatgtttgt tatcttcttt ttctgttcac tcagaggtca tcaatttctt tctctccgtc     4320
   cttaattgga ttatttttct tttggccttt gggcacagag tctgacctct ggaccactct     4380
   aactggagaa ggaactttat gttccctctc ctgctgtgtc cacaacctta gaaatctgta     4440
   gctagatttt tgttgttata gatagaattt actgtttctg aaacccaaat acagttatca     4500
   gtttaaggtt                                                            4510
<210> 14
   <211> 1189
   <212> PRT
   <213> Homo sapiens
<220>
   <221> misc_feature
   <223> amino acid sequence of the TDRD1 gene (NM_198795.1, NP_942090.1)
<400> 14
   Met Ser Val Lys Ser Pro Phe Asn Val Met Ser Arg Asn Asn Leu Glu
   1               5                   10                  15
   Ala Pro Pro Cys Lys Met Thr Glu Pro Phe Asn Phe Glu Lys Asn Glu
               20                  25                  30
   Asn Lys Leu Pro Pro His Glu Ser Leu Arg Ser Pro Gly Thr Leu Pro
   35 40 45
   Asn His Pro Asn Phe Arg Leu Lys Ser Ser Glu Asn Gly Asn Lys Lys
   50 55 60
   Asn Asn Phe Leu Leu Cys Glu Gln Thr Lys Gln Tyr Leu Ala Ser Gln
   65 70 75 80
   Glu Asp Asn Ser Val Ser Ser Asn Pro Asn Gly Ile Asn Gly Glu Val
   85 90 95
   Val Gly Ser Lys Gly Asp Arg Lys Lys Leu Pro Ala Gly Asn Ser Val
   100 105 110
   Ser Pro Pro Ser Ala Glu Ser Asn Ser Pro Pro Lys Glu Val Asn Ile
   115 120 125
   Lys Pro Gly Asn Asn Val Arg Pro Ala Lys Ser Lys Lys Leu Asn Lys
   130 135 140
   Leu Val Glu Asn Ser Leu Ser Ile Ser Asn Pro Gly Leu Phe Thr Ser
   145 150 155 160
   Leu Gly Pro Pro Leu Arg Ser Thr Thr Cys His Arg Cys Gly Leu Phe
   165 170 175
   Gly Ser Leu Arg Cys Ser Gln Cys Lys Gln Thr Tyr Tyr Cys Ser Thr
   180 185 190
   Ala Cys Gln Arg Arg Asp Trp Ser Ala His Ser Ile Val Cys Arg Pro
   195 200 205
   Val Gln Pro Asn Phe His Lys Leu Glu Asn Lys Ser Ser Ile Glu Thr
   210 215 220
   Lys Asp Val Glu Val Asn Asn Lys Ser Asp Cys Pro Leu Gly Val Thr
   225 230 235 240
   Lys Glu Ile Ala Ile Trp Ala Glu Arg Ile Met Phe Ser Asp Leu Arg
   245 250 255
   Ser Leu Gln Leu Lys Lys Thr Met Glu Ile Lys Gly Thr Val Thr Glu
   260 265 270
   Phe Lys His Pro Gly Asp Phe Tyr Val Gln Leu Tyr Ser Ser Glu Val
   275 280 285
   Leu Glu Tyr Met Asn Gln Leu Ser Ala Ser Leu Lys Glu Thr Tyr Ala
   290 295 300
   Asn Val His Glu Lys Asp Tyr Ile Pro Val Lys Gly Glu Val Cys Ile
   305 310 315 320
   Ala Lys Tyr Thr Val Asp Gln Thr Trp Asn Arg Ala Ile Ile Gln Asn
   325 330 335
   Val Asp Val Gln Gln Lys Lys Ala His Val Leu Tyr Ile Asp Tyr Gly
   340 345 350
   Asn Glu Glu Ile Ile Pro Leu Asn Arg Ile Tyr His Leu Asn Arg Asn
   355 360 365
   Ile Asp Leu Phe Pro Pro Cys Ala Ile Lys Cys Phe Val Ala Asn Val
   370 375 380
   Ile Pro Ala Glu Gly Asn Trp Ser Ser Asp Cys Ile Lys Ala Thr Lys
   385 390 395 400
   Pro Leu Leu Met Glu Gln Tyr Cys Ser Ile Lys Ile Val Asp Ile Leu
   405 410 415
   Glu Glu Glu Val Val Thr Phe Ala Val Glu Val Glu Leu Pro Asn Ser
   420 425 430
   Gly Lys Leu Leu Asp His Val Leu Ile Glu Met Gly Tyr Gly Leu Lys
   435 440 445
   Pro Ser Gly Gln Asp Ser Lys Lys Glu Asn Ala Asp Gln Ser Asp Pro
       450                 455                 460
   Glu Asp Val Gly Lys Met Thr Thr Glu Asn Asn Ile Val Val Asp Lys
   465                 470                 475                 480
   Ser Asp Leu Ile Pro Lys Val Leu Thr Leu Asn Val Gly Asp Glu Phe
                   485                 490                 495
   Cys Gly Val Val Ala His Ile Gln Thr Pro Glu Asp Phe Phe Cys Gln
               500                 505                 510
   Gln Leu Gln Ser Gly Arg Lys Leu Ala Glu Leu Gln Ala Ser Leu Ser
           515                 520                 525
   Lys Tyr Cys Asp Gln Leu Pro Pro Arg Ser Asp Phe Tyr Pro Ala Ile
       530                 535                 540
   Gly Asp Ile Cys Cys Ala Gln Phe Ser Glu Asp Asp Gln Trp Tyr Arg
   545                 550                 555                 560
   Ala Ser Val Leu Ala Tyr Ala Ser Glu Glu Ser Val Leu Val Gly Tyr
                   565                 570                 575
   Val Asp Tyr Gly Asn Phe Glu Ile Leu Ser Leu Met Arg Leu Cys Pro
               580                 585                 590
   Ile Ile Pro Lys Leu Leu Glu Leu Pro Met Gln Ala Ile Lys Cys Val
           595                 600                 605
   Leu Ala Gly Val Lys Pro Ser Leu Gly Ile Trp Thr Pro Glu Ala Ile
       610                 615                 620
   Cys Leu Met Lys Lys Leu Val Gln Asn Lys Ile Ile Thr Val Lys Val
   625                 630                 635                 640
   Val Asp Lys Leu Glu Asn Ser Ser Leu Val Glu Leu Ile Asp Lys Ser
                   645                 650                 655
   Glu Thr Pro His Val Ser Val Ser Lys Val Leu Leu Asp Ala Gly Phe
               660                 665                 670
   Ala Val Gly Glu Gln Ser Met Val Thr Asp Lys Pro Ser Asp Val Lys
           675                 680                 685
   Glu Thr Ser Val Pro Leu Gly Val Glu Gly Lys Val Asn Pro Leu Glu
       690                 695                 700
   Trp Thr Trp Val Glu Leu Gly Val Asp Gln Thr Val Asp Val Val Val
   705                 710                 715                 720
   Cys Val Ile Tyr Ser Pro Gly Glu Phe Tyr Cys His Val Leu Lys Glu
                   725                 730                 735
   Asp Ala Leu Lys Lys Leu Asn Asp Leu Asn Lys Ser Leu Ala Glu His
               740                 745                 750
   Cys Gln Gln Lys Leu Pro Asn Gly Phe Lys Ala Glu Ile Gly Gln Pro
           755                 760                 765
   Cys Cys Ala Phe Phe Ala Gly Asp Gly Ser Trp Tyr Arg Ala Leu Val
       770                 775                 780
   Lys Glu Ile Leu Pro Asn Gly His Val Lys Val His Phe Val Asp Tyr
   785                 790                 795                 800
   Gly Asn Ile Glu Glu Val Thr Ala Asp Glu Leu Arg Met Ile Ser Ser
                   805                 810                 815
   Thr Phe Leu Asn Leu Pro Phe Gln Gly Ile Arg Cys Gln Leu Ala Asp
               820                 825                 830
   Ile Gln Ser Arg Asn Lys His Trp Ser Glu Glu Ala Ile Thr Arg Phe
           835                 840                 845
   Gln Met Cys Val Ala Gly Ile Lys Leu Gln Ala Arg Val Val Glu Val
       850                 855                 860
   Thr Glu Asn Gly Ile Gly Val Glu Leu Thr Asp Leu Ser Thr Cys Tyr
   865                 870                 875                 880
   Pro Arg Ile Ile Ser Asp Val Leu Ile Asp Glu His Leu Val Leu Lys
                   885                 890                 895
   Ser Ala Ser Pro His Lys Asp Leu Pro Asn Asp Arg Leu Val Asn Lys
               900                 905                 910
   His Glu Leu Gln Val His Val Gln Gly Leu Gln Ala Thr Ser Ser Ala
           915                 920                 925
   Glu Gln Trp Lys Thr Ile Glu Leu Pro Val Asp Lys Thr Ile Gln Ala
       930                 935                 940
   Asn Val Leu Glu Ile Ile Ser Pro Asn Leu Phe Tyr Ala Leu Pro Lys
   945                 950                 955                 960
   Gly Met Pro Glu Asn Gln Glu Lys Leu Cys Met Leu Thr Ala Glu Leu
                   965                 970                 975
   Leu Glu Tyr Cys Asn Ala Pro Lys Ser Arg Pro Pro Tyr Arg Pro Arg
               980                 985                 990
   Ile Gly Asp Ala Cys Cys Ala Lys  Tyr Thr Ser Asp Asp  Phe Trp Tyr
           995                 1000                 1005
   Arg Ala  Val Val Leu Gly Thr  Ser Asp Thr Asp Val  Glu Val Leu
       1010                 1015                 1020
   Tyr Ala  Asp Tyr Gly Asn Ile  Glu Thr Leu Pro Leu  Cys Arg Val
       1025                 1030                 1035
   Gln Pro  Ile Thr Ser Ser His  Leu Ala Leu Pro Phe  Gln Ile Ile
       1040                 1045                 1050
   Arg Cys  Ser Leu Glu Gly Leu  Met Glu Leu Asn Gly  Ser Ser Ser
       1055                 1060                 1065
   Gln Leu  Ile Ile Met Leu Leu  Lys Asn Phe Met Leu  Asn Gln Asn
       1070                 1075                 1080
   Val Met  Leu Ser Val Lys Gly  Ile Thr Lys Asn Val  His Thr Val
       1085                 1090                 1095
   Ser Val  Glu Lys Cys Ser Glu  Asn Gly Thr Val Asp  Val Ala Asp
       1100                 1105                 1110
   Lys Leu  Val Thr Phe Gly Leu  Ala Lys Asn Ile Thr  Pro Gln Arg
       1115                 1120                 1125
   Gln Ser  Ala Leu Asn Thr Glu  Lys Met Tyr Arg Met  Asn Cys Cys
       1130                 1135                 1140
   Cys Thr  Glu Leu Gln Lys Gln  Val Glu Lys His Glu  His Ile Leu
       1145                 1150                 1155
   Leu Phe  Leu Leu Asn Asn Ser  Thr Asn Gln Asn Lys  Phe Ile Glu
       1160                 1165                 1170
   Met Lys  Lys Leu Leu Lys Lys  Thr Ala Ser Leu Gly  Gly Lys Pro
       1175                 1180                 1185
   Leu

### SEQUENCE LISTING

<110> NovioGendix Research B.V.
<120> COMBINATIONS OF MOLECULAR MARKERS IN PROSTATE CANCER PROVIDING A DIAGNOSTIC TOOL WITH IMPROVED SENSITIVITY/SPECIFICITY
<130> 4/2QK29/12P
<150> PCT/EP2013/066889
   <151> 2013-08-13
<160> 14
<170> PatentIn version 3.5
<210> 1
   <211> 2300
   <212> DNA
   <213> Homo sapiens
<220>
   <221> misc_feature
   <223> cDNA sequence of the variant 1 of the HOXC4 gene (NM_014620.4, NP_055435.2)
<400> 1
   ttattgtggt ttgtccgttc cgagcgctcc gcagaacagt cctccctgta agagcctaac      60
   cattgccagg gaaacctgcc ctgggcgctc ccttcattag cagtattttt tttaaattaa     120
   tctgattaat aattattttt cccccattta attttttttc ctcccaggtg gagttgccga     180
   agctgggggc agctggggag ggtggggatg ggaggggaga gacagaagtt gagggcatct     240
   ctctcttcct tcccgaccct ctggccccca aggggcagga ggaatgcagg agcaggagtt     300
   gagcttggga gctgcagatg cctccgcccc tcctctctcc caggctcttc ctcctgcccc     360
   cttcttgcaa ctctccttaa ttttgtttgg cttttggatg attataatta tttttatttt     420
   tgaatttata taaagtatat gtgtgtgtgt gtggagctga gacaggctcg gcagcggcac     480
   agaatgaggg aagacgagaa agagagtggg agagagagag gcagagaggg agagagggag     540
   agtgacagca gcgctcgcgg gggctcaacc cccagacctc cagaaatgac gtcagaatca     600
   tttgcatccc gctgcctcta cctgcctggt ccagctggga ccctgcctcg ccggccgcat     660
   ggccagaggg ttggaaatta atgatcatga gctcgtattt gatggactct aactacatcg     720
   atccgaaatt tcctccatgc gaagaatatt cgcaaaatag ctacatccct gaacacagtc     780
   cggaatatta cggccggacc agggaatcgg gattccagca tcaccaccag gagctgtacc     840
   caccaccgcc tccgcgccct agctaccctg agcgccagta tagctgcacc agtctccagg     900
   ggcccggcaa ttcgcgaggc cacgggccgg cccaggcggg ccaccaccac cccgagaaat     960
   cacagtcgct ctgcgagccg gcgcctctct caggcgcctc cgcctccccg tccccagccc    1020
   cgccagcctg cagccagcca gcccccgacc atccctccag cgccgccagc aagcaaccca    1080
   tagtctaccc atggatgaaa aaaattcacg ttagcacggt gaaccccaat tataacggag    1140
   gggaacccaa gcgctcgagg acagcctata cccggcagca agtcctggaa ttagagaaag    1200
   agtttcatta caaccgctac ctgacccgaa ggagaaggat cgagatcgcc cactcgctgt    1260
   gcctctctga gaggcagatc aaaatctggt tccaaaaccg tcgcatgaaa tggaagaagg    1320
   accaccgact ccccaacacc aaagtcaggt cagcaccccc ggccggcgct gcgcccagca    1380
   ccctttcggc agctaccccg ggtacttctg aagaccactc ccagagcgcc acgccgccgg    1440
   agcagcaacg ggcagaggac attaccaggt tataaaacat aactcacacc cctgccccca    1500
   ccccatgccc ccaccctccc ctcacacaca aattgactct tatttataga atttaatata    1560
   tatatatata tatatatata taggttcttt tctctcttcc tctcaccttg tcccttgtca     1620
   gttccaaaca gacaaaacag ataaacaaac aagccccctg ccctcctctc cctcccactg     1680
   ttaaggaccc ttttaagcat gtgatgttgt cttagcatgg tacctgctgg gtgttttttt     1740
   ttaaaaggcc attttggggg gttatttatt ttttaagaaa aaaagctgca aaaattatat     1800
   attgcaaggt gtgatggtct ggcttgggtg aatttcaggg gaaatgagga aaagaaaaaa     1860
   ggaaagaaat tttaaagcca attctcatcc ttctcctcct cctccttccc cccctctttc     1920
   cttaggcctt ttgcattgaa aatgcaccag gggaggttag tgagggggaa gtcattttaa     1980
   ggagaacaaa gctatgaagt tcttttgtat tattgttggg ggggggtgtg ggaggagagg     2040
   gggcgaagac agcagacaaa gctaaatgca tctggagagc ctctcagagc tgttcagttt     2100
   gaggagccaa aagaaaatca aaatgaactt tcagttcaga gaggcagtct ataggtagaa     2160
   tctctcccca cccctatcgt ggttattgtg tttttggact gaatttactt gattattgta     2220
   aaacttgcaa taaagaattt tagtgtcgat gtgaaatgcc ccgtgatcaa taataaacca     2280
   gtggatgtga attagtttta                                                 2300
<210> 2
   <211> 264
   <212> PRT
   <213> Homo sapiens
<220>
   <221> misc_feature
   <223> amino acid sequence of the variant 1 of the HOXC4 gene (NM_014620.4, NP_055435.2)
<400> 2
   Met Ile Met Ser Ser Tyr Leu Met Asp Ser Asn Tyr Ile Asp Pro Lys
   1               5                   10                  15
   Phe Pro Pro Cys Glu Glu Tyr Ser Gln Asn Ser Tyr Ile Pro Glu His
               20                  25                  30
   Ser Pro Glu Tyr Tyr Gly Arg Thr Arg Glu Ser Gly Phe Gln His His
           35                  40                  45
   His Gln Glu Leu Tyr Pro Pro Pro Pro Pro Arg Pro Ser Tyr Pro Glu
       50                  55                  60
   Arg Gln Tyr Ser Cys Thr Ser Leu Gln Gly Pro Gly Asn Ser Arg Gly
   65                  70                  75                  80
   His Gly Pro Ala Gln Ala Gly His His His Pro Glu Lys Ser Gln Ser
                   85                  90                  95
   Leu Cys Glu Pro Ala Pro Leu Ser Gly Ala Ser Ala Ser Pro Ser Pro
               100                 105                 110
   Ala Pro Pro Ala Cys Ser Gln Pro Ala Pro Asp His Pro Ser Ser Ala
           115                 120                 125
   Ala Ser Lys Gln Pro Ile Val Tyr Pro Trp Met Lys Lys Ile His Val
       130                 135                 140
   Ser Thr Val Asn Pro Asn Tyr Asn Gly Gly Glu Pro Lys Arg Ser Arg
   145                 150                 155                 160
   Thr Ala Tyr Thr Arg Gln Gln Val Leu Glu Leu Glu Lys Glu Phe His
                   165                 170                 175
   Tyr Asn Arg Tyr Leu Thr Arg Arg Arg Arg Ile Glu Ile Ala His Ser
               180                 185                 190
   Leu Cys Leu Ser Glu Arg Gln Ile Lys Ile Trp Phe Gln Asn Arg Arg
           195                 200                 205
   Met Lys Trp Lys Lys Asp His Arg Leu Pro Asn Thr Lys Val Arg Ser
       210                 215                 220
   Ala Pro Pro Ala Gly Ala Ala Pro Ser Thr Leu Ser Ala Ala Thr Pro
   225                 230                 235                 240
   Gly Thr Ser Glu Asp His Ser Gln Ser Ala Thr Pro Pro Glu Gln Gln
                   245                 250                 255
   Arg Ala Glu Asp Ile Thr Arg Leu
               260
<210> 3
   <211> 1689
   <212> DNA
   <213> Homo sapiens
<220>
   <221> misc_feature
   <223> cDNA sequence of the variant 2 of the HOXC4 gene (NM_153633.2, NP_705897.1)
<400> 3
   agaaaaacga caaagcgaga aaaattattt tccactccag aaattaatga tcatgagctc      60
   gtatttgatg gactctaact acatcgatcc gaaatttcct ccatgcgaag aatattcgca     120
   aaatagctac atccctgaac acagtccgga atattacggc cggaccaggg aatcgggatt     180
   ccagcatcac caccaggagc tgtacccacc accgcctccg cgccctagct accctgagcg     240
   ccagtatagc tgcaccagtc tccaggggcc cggcaattcg cgaggccacg ggccggccca     300
   ggcgggccac caccaccccg agaaatcaca gtcgctctgc gagccggcgc ctctctcagg     360
   cgcctccgcc tccccgtccc cagccccgcc agcctgcagc cagccagccc ccgaccatcc     420
   ctccagcgcc gccagcaagc aacccatagt ctacccatgg atgaaaaaaa ttcacgttag     480
   cacggtgaac cccaattata acggagggga acccaagcgc tcgaggacag cctatacccg     540
   gcagcaagtc ctggaattag agaaagagtt tcattacaac cgctacctga cccgaaggag     600
   aaggatcgag atcgcccact cgctgtgcct ctctgagagg cagatcaaaa tctggttcca     660
   aaaccgtcgc atgaaatgga agaaggacca ccgactcccc aacaccaaag tcaggtcagc     720
   acccccggcc ggcgctgcgc ccagcaccct ttcggcagct accccgggta cttctgaaga     780
   ccactcccag agcgccacgc cgccggagca gcaacgggca gaggacatta ccaggttata     840
   aaacataact cacacccctg cccccacccc atgcccccac cctcccctca cacacaaatt     900
   gactcttatt tatagaattt aatatatata tatatatata tatatatagg ttcttttctc     960
   tcttcctctc accttgtccc ttgtcagttc caaacagaca aaacagataa acaaacaagc    1020
   cccctgccct cctctccctc ccactgttaa ggaccctttt aagcatgtga tgttgtctta    1080
   gcatggtacc tgctgggtgt ttttttttaa aaggccattt tggggggtta tttatttttt    1140
   aagaaaaaaa gctgcaaaaa ttatatattg caaggtgtga tggtctggct tgggtgaatt    1200
   tcaggggaaa tgaggaaaag aaaaaaggaa agaaatttta aagccaattc tcatccttct    1260
   cctcctcctc cttccccccc tctttcctta ggccttttgc attgaaaatg caccagggga    1320
   ggttagtgag ggggaagtca ttttaaggag aacaaagcta tgaagttctt ttgtattatt    1380
   gttggggggg ggtgtgggag gagagggggc gaagacagca gacaaagcta aatgcatctg    1440
   gagagcctct cagagctgtt cagtttgagg agccaaaaga aaatcaaaat gaactttcag    1500
   ttcagagagg cagtctatag gtagaatctc tccccacccc tatcgtggtt attgtgtttt    1560
   tggactgaat ttacttgatt attgtaaaac ttgcaataaa gaattttagt gtcgatgtga    1620
   aatgccccgt gatcaataat aaaccagtgg atgtgaatta gttttaaaaa aaaaaaaaaa    1680
   aaaaaaaaa                                                            1689
<210> 4
   <211> 264
   <212> PRT
   <213> Homo sapiens
<220>
   <221> misc_feature
   <223> amino acid sequence of the variant 2 of the HOXC4 gene (NM_153633.2, NP_705897.1)
<400> 4
   Met Ile Met Ser Ser Tyr Leu Met Asp Ser Asn Tyr Ile Asp Pro Lys
   1               5                   10                  15
   Phe Pro Pro Cys Glu Glu Tyr Ser Gln Asn Ser Tyr Ile Pro Glu His
               20                  25                  30
   Ser Pro Glu Tyr Tyr Gly Arg Thr Arg Glu Ser Gly Phe Gln His His
           35                  40                  45
   His Gln Glu Leu Tyr Pro Pro Pro Pro Pro Arg Pro Ser Tyr Pro Glu
       50                  55                  60
   Arg Gln Tyr Ser Cys Thr Ser Leu Gln Gly Pro Gly Asn Ser Arg Gly
   65                  70                  75                  80
   His Gly Pro Ala Gln Ala Gly His His His Pro Glu Lys Ser Gln Ser
                   85                  90                  95
   Leu Cys Glu Pro Ala Pro Leu Ser Gly Ala Ser Ala Ser Pro Ser Pro
               100                 105                 110
   Ala Pro Pro Ala Cys Ser Gln Pro Ala Pro Asp His Pro Ser Ser Ala
           115                 120                 125
   Ala Ser Lys Gln Pro Ile Val Tyr Pro Trp Met Lys Lys Ile His Val
       130                 135                 140
   Ser Thr Val Asn Pro Asn Tyr Asn Gly Gly Glu Pro Lys Arg Ser Arg
   145                 150                 155                 160
   Thr Ala Tyr Thr Arg Gln Gln Val Leu Glu Leu Glu Lys Glu Phe His
                   165                 170                 175
   Tyr Asn Arg Tyr Leu Thr Arg Arg Arg Arg Ile Glu Ile Ala His Ser
               180                 185                 190
   Leu Cys Leu Ser Glu Arg Gln Ile Lys Ile Trp Phe Gln Asn Arg Arg
           195                 200                 205
   Met Lys Trp Lys Lys Asp His Arg Leu Pro Asn Thr Lys Val Arg Ser
       210                 215                 220
   Ala Pro Pro Ala Gly Ala Ala Pro Ser Thr Leu Ser Ala Ala Thr Pro
   225                 230                 235                 240
   Gly Thr Ser Glu Asp His Ser Gln Ser Ala Thr Pro Pro Glu Gln Gln
                   245                 250                 255
   Arg Ala Glu Asp Ile Thr Arg Leu
               260
<210> 5
   <211> 1681
   <212> DNA
   <213> Homo sapiens
<220>
   <221> misc_feature
   <223> cDNA sequence of the variant 1 of the HOXC6 gene (NM_004503.3, NP_004494.1)
<400> 5
   ttttgtctgt cctggattgg agccgtccct ataaccatct agttccgagt acaaactgga      60
   gacagaaata aatattaaag aaatcataga ccgaccaggt aaaggcaaag ggatgaattc     120
   ctacttcact aacccttcct tatcctgcca cctcgccggg ggccaggacg tcctccccaa     180
   cgtcgccctc aattccaccg cctatgatcc agtgaggcat ttctcgacct atggagcggc      240
   cgttgcccag aaccggatct actcgactcc cttttattcg ccacaggaga atgtcgtgtt      300
   cagttccagc cgggggccgt atgactatgg atctaattcc ttttaccagg agaaagacat      360
   gctctcaaac tgcagacaaa acaccttagg acataacaca cagacctcaa tcgctcagga      420
   ttttagttct gagcagggca ggactgcgcc ccaggaccag aaagccagta tccagattta      480
   cccctggatg cagcgaatga attcgcacag tggggtcggc tacggagcgg accggaggcg      540
   cggccgccag atctactcgc ggtaccagac cctggaactg gagaaggaat ttcacttcaa      600
   tcgctaccta acgcggcgcc ggcgcatcga gatcgccaac gcgctttgcc tgaccgagcg      660
   acagatcaaa atctggttcc agaaccgccg gatgaagtgg aaaaaagaat ctaatctcac      720
   atccactctc tcggggggcg gcggaggggc caccgccgac agcctgggcg gaaaagagga      780
   aaagcgggaa gagacagaag aggagaagca gaaagagtga ccaggactgt ccctgccacc      840
   cctctctccc tttctccctc gctccccacc aactctcccc taatcacaca ctctgtattt      900
   atcactggca caattgatgt gttttgattc cctaaaacaa aattagggag tcaaacgtgg      960
   acctgaaagt cagctctgga ccccctccct caccgcacaa ctctctttca ccacgcgcct     1020
   cctcctcctc gctcccttgc tagctcgttc tcggcttgtc tacaggccct tttccccgtc     1080
   caggccttgg gggctcggac cctgaactca gactctacag attgccctcc aagtgaggac     1140
   ttggctcccc cactccttcg acgcccccac ccccgccccc cgtgcagaga gccggctcct     1200
   gggcctgctg gggcctctgc tccagggcct cagggcccgg cctggcagcc ggggagggcc     1260
   ggaggcccaa ggagggcgcg ccttggcccc acaccaaccc ccagggcctc cccgcagtcc     1320
   ctgcctagcc cctctgcccc agcaaatgcc cagcccaggc aaattgtatt taaagaatcc     1380
   tgggggtcat tatggcattt tacaaactgt gaccgtttct gtgtgaagat ttttagctgt     1440
   atttgtggtc tctgtattta tatttatgtt tagcaccgtc agtgttccta tccaatttca     1500
   aaaaaggaaa aaaaagaggg aaaattacaa aaagagagaa aaaaagtgaa tgacgtttgt     1560
   ttagccagta ggagaaaata aataaataaa taaatccctt cgtgttaccc tcctgtataa     1620
   atccaacctc tgggtccgtt ctcgaatatt taataaaact gatattattt ttaaaacttt     1680
   a                                                                     1681
<210> 6
   <211> 235
   <212> PRT
   <213> Homo sapiens
<220>
   <221> misc_feature
   <223> amino acid sequence of the variant 1 of the HOXC6 gene (NM_004503.3, NP_004494.1)
<400> 6
   Met Asn Ser Tyr Phe Thr Asn Pro Ser Leu Ser Cys His Leu Ala Gly
   1               5                   10                  15
   Gly Gln Asp Val Leu Pro Asn Val Ala Leu Asn Ser Thr Ala Tyr Asp
               20                  25                  30
   Pro Val Arg His Phe Ser Thr Tyr Gly Ala Ala Val Ala Gln Asn Arg
           35                  40                  45
   Ile Tyr Ser Thr Pro Phe Tyr Ser Pro Gln Glu Asn Val Val Phe Ser
       50                  55                  60
   Ser Ser Arg Gly Pro Tyr Asp Tyr Gly Ser Asn Ser Phe Tyr Gln Glu
   65                  70                  75                  80
   Lys Asp Met Leu Ser Asn Cys Arg Gln Asn Thr Leu Gly His Asn Thr
                   85                  90                  95
   Gln Thr Ser Ile Ala Gln Asp Phe Ser Ser Glu Gln Gly Arg Thr Ala
               100                 105                 110
   Pro Gln Asp Gln Lys Ala Ser Ile Gln Ile Tyr Pro Trp Met Gln Arg
           115                 120                 125
   Met Asn Ser His Ser Gly Val Gly Tyr Gly Ala Asp Arg Arg Arg Gly
       130                 135                 140
   Arg Gln Ile Tyr Ser Arg Tyr Gln Thr Leu Glu Leu Glu Lys Glu Phe
   145                 150                 155                 160
   His Phe Asn Arg Tyr Leu Thr Arg Arg Arg Arg Ile Glu Ile Ala Asn
                   165                 170                 175
   Ala Leu Cys Leu Thr Glu Arg Gln Ile Lys Ile Trp Phe Gln Asn Arg
               180                 185                 190
   Arg Met Lys Trp Lys Lys Glu Ser Asn Leu Thr Ser Thr Leu Ser Gly
           195                 200                 205
   Gly Gly Gly Gly Ala Thr Ala Asp Ser Leu Gly Gly Lys Glu Glu Lys
       210                 215                 220
   Arg Glu Glu Thr Glu Glu Glu Lys Gln Lys Glu
   225                 230                 235
<210> 7
   <211> 2072
   <212> DNA
   <213> Homo sapiens
<220>
   <221> misc_feature
   <223> cDNA sequence of the variant 2 of the HOXC6 gene (NM_153693.3, NP_710160.1)
<400> 7
   ttattgtggt ttgtccgttc cgagcgctcc gcagaacagt cctccctgta agagcctaac      60
   cattgccagg gaaacctgcc ctgggcgctc ccttcattag cagtattttt tttaaattaa     120
   tctgattaat aattattttt cccccattta attttttttc ctcccaggtg gagttgccga      180
   agctgggggc agctggggag ggtggggatg ggaggggaga gacagaagtt gagggcatct     240
   ctctcttcct tcccgaccct ctggccccca aggggcagga ggaatgcagg agcaggagtt     300
   gagcttggga gctgcagatg cctccgcccc tcctctctcc caggctcttc ctcctgcccc     360
   cttcttgcaa ctctccttaa ttttgtttgg cttttggatg attataatta tttttatttt     420
   tgaatttata taaagtatat gtgtgtgtgt gtggagctga gacaggctcg gcagcggcac     480
   agaatgaggg aagacgagaa agagagtggg agagagagag gcagagaggg agagagggag     540
   agtgacagca gcgctcggac gtcctcccca acgtcgccct caattccacc gcctatgatc     600
   cagtgaggca tttctcgacc tatggagcgg ccgttgccca gaaccggatc tactcgactc     660
   ccttttattc gccacaggag aatgtcgtgt tcagttccag ccgggggccg tatgactatg     720
   gatctaattc cttttaccag gagaaagaca tgctctcaaa ctgcagacaa aacaccttag     780
   gacataacac acagacctca atcgctcagg attttagttc tgagcagggc aggactgcgc     840
   cccaggacca gaaagccagt atccagattt acccctggat gcagcgaatg aattcgcaca     900
   gtggggtcgg ctacggagcg gaccggaggc gcggccgcca gatctactcg cggtaccaga     960
   ccctggaact ggagaaggaa tttcacttca atcgctacct aacgcggcgc cggcgcatcg    1020
   agatcgccaa cgcgctttgc ctgaccgagc gacagatcaa aatctggttc cagaaccgcc    1080
   ggatgaagtg gaaaaaagaa tctaatctca catccactct ctcggggggc ggcggagggg     1140
   ccaccgccga cagcctgggc ggaaaagagg aaaagcggga agagacagaa gaggagaagc    1200
   agaaagagtg accaggactg tccctgccac ccctctctcc ctttctccct cgctccccac    1260
   caactctccc ctaatcacac actctgtatt tatcactggc acaattgatg tgttttgatt    1320
   ccctaaaaca aaattaggga gtcaaacgtg gacctgaaag tcagctctgg accccctccc    1380
   tcaccgcaca actctctttc accacgcgcc tcctcctcct cgctcccttg ctagctcgtt    1440
   ctcggcttgt ctacaggccc ttttccccgt ccaggccttg ggggctcgga ccctgaactc     1500
   agactctaca gattgccctc caagtgagga cttggctccc ccactccttc gacgccccca    1560
   cccccgcccc ccgtgcagag agccggctcc tgggcctgct ggggcctctg ctccagggcc     1620
   tcagggcccg gcctggcagc cggggagggc cggaggccca aggagggcgc gccttggccc     1680
   cacaccaacc cccagggcct ccccgcagtc cctgcctagc ccctctgccc cagcaaatgc     1740
   ccagcccagg caaattgtat ttaaagaatc ctgggggtca ttatggcatt ttacaaactg     1800
   tgaccgtttc tgtgtgaaga tttttagctg tatttgtggt ctctgtattt atatttatgt     1860
   ttagcaccgt cagtgttcct atccaatttc aaaaaaggaa aaaaaagagg gaaaattaca     1920
   aaaagagaga aaaaaagtga atgacgtttg tttagccagt aggagaaaat aaataaataa     1980
   ataaatccct tcgtgttacc ctcctgtata aatccaacct ctgggtccgt tctcgaatat     2040
   ttaataaaac tgatattatt tttaaaactt ta                                   2072
<210> 8
   <211> 153
   <212> PRT
   <213> Homo sapiens
<220>
   <221> misc_feature
   <223> mino acid sequence of the variant 2 of the HOXC6 gene (NM_153693.3, NP_710160.1)
<400> 8
   Met Leu Ser Asn Cys Arg Gln Asn Thr Leu Gly His Asn Thr Gln Thr
   1               5                   10                  15
   Ser Ile Ala Gln Asp Phe Ser Ser Glu Gln Gly Arg Thr Ala Pro Gln
               20                  25                  30
   Asp Gln Lys Ala Ser Ile Gln Ile Tyr Pro Trp Met Gln Arg Met Asn
           35                  40                  45
   Ser His Ser Gly Val Gly Tyr Gly Ala Asp Arg Arg Arg Gly Arg Gln
       50                  55                  60
   Ile Tyr Ser Arg Tyr Gln Thr Leu Glu Leu Glu Lys Glu Phe His Phe
   65                  70                  75                  80
   Asn Arg Tyr Leu Thr Arg Arg Arg Arg Ile Glu Ile Ala Asn Ala Leu
                   85                  90                  95
   Cys Leu Thr Glu Arg Gln Ile Lys Ile Trp Phe Gln Asn Arg Arg Met
               100                 105                 110
   Lys Trp Lys Lys Glu Ser Asn Leu Thr Ser Thr Leu Ser Gly Gly Gly
           115                 120                 125
   Gly Gly Ala Thr Ala Asp Ser Leu Gly Gly Lys Glu Glu Lys Arg Glu
       130                 135                 140
   Glu Thr Glu Glu Glu Lys Gln Lys Glu
   145                 150
<210> 9
   <211> 2403
   <212> DNA
   <213> Homo sapiens
<220>
   <221> misc_feature
   <223> cDNA sequence of transcript variant one of the DLX1 gene (NM_178120, NP_835221)
<400> 9
   aagctttgaa ccgagtttgg ggagctcagc agcatcatgc ttagactttt caaagagaca       60
   aactccattt tcttatgaat ggaaagtgaa aacccctgtt ccgcttaaat tgggttcctt      120
   cctgtcctga gaaacataga gacccccaaa agggaagcag aggagagaaa gtcccacacc 180
   cagaccccgc gagaagagat gaccatgacc accatgccag aaagtctcaa cagccccgtg 240
   tcgggcaagg cggtgtttat ggagtttggg ccgcccaacc agcaaatgtc tccttctccc 300
   atgtcccacg ggcactactc catgcactgt ttacactcgg cgggccattc gcagcccgac 360
   ggcgcctaca gctcagcctc gtccttctcc cgaccgctgg gctaccccta cgtcaactcg 420
   gtcagcagcc acgcatccag cccctacatc agttcggtgc agtcctaccc gggcagcgcc 480
   agcctcgccc agagccgcct ggaggaccca ggggcggact cggagaagag cacggtggtg 540
   gaaggcggtg aagtgcgctt caatggcaag ggaaaaaaga tccgtaaacc caggacgatt 600
   tattccagtt tgcagttgca ggctttgaac cggaggttcc agcaaactca gtacctagct 660
   ctgccggaga gggcggagct cgcggcctct ttgggactca cacagactca ggtcaagatc 720
   tggttccaaa acaagcgatc caagttcaag aagctgatga agcagggtgg ggcggctctg 780
   gagggtagtg cgttggccaa cggtcgggcc ctgtctgctg gctccccacc cgtgccgccc 840
   ggctggaacc ctaactcttc atccgggaag ggctcaggag gaaacgcggg ctcctatatc 900
   cccagctaca catcgtggta cccttcagcg caccaagaag ctatgcagca accccaactt 960
   atgtgaggtt gcccgcccgt ctccttcttg tctccccggc ccaggtccct cccgcctcca 1020
   ggtccatcca tcccgtccgg aaaagaagga cccagaggga agaaggaaca gtggaggcgg 1080
   gacgccctcc atctcctcgg agccccgcga ggtccggccc agcaacttcc cggcatccgc 1140
   gctctagcct gaaccctggc ctgggccgag cagtggcagc agagagtggc ctcggaggga 1200
   agccactgcc acctgagaca gcccaagcag caagataaac ccgctccacc cgacccgccg 1260
   accttcagct ttgtgggact atcaggaaaa aacaaaacaa aaacaaaatg tagaaaaagc 1320
   aaaagctctt ttctgtcctg tcagtctcct gtctcctttt gctctgtctg tgcgctggta 1380
   aagtccaggt cctcatccgt ccgctgtcct cattctgcgg cctcagcaaa aagccacaag 1440
   gtctgagcgg cccgggtcct gccgggctga ccatctccgg atcctgggac actctgcctg 1500
   accatctgtg tagctggtgt gggaatctgg gggcattgga gggagggggt tttatttatt 1560
   gagaaatgga cttcgcctga ggctgtttgc caattcaggg ttctgctggg cgcaaggaac 1620
   gcactgttca aacgcactgt ttactttaag cgcacgggga gaaacgaata aggaggacgt 1680
   ggtgattttt aatttataca gtaacttttg tacttctctg gtatggagag tttggagccg 1740
   aatgatttgc attttttaca tgtccgacat tatttaataa ataattttta aaagaaaaga 1800
   acgataaatg aagccaacat gattttctca tttcgggagg aactctgttg cttcgcctgg 1860
   acaagaagga aaatgctgat ttcctccttg ggtagaaaga gggagcgagg gcaaatgggg 1920
   agtagagaga aaacaggcga gaacaagcac tctaattcca gtgggcttta aaataagaca 1980
   aaatcagctt tacaacaatc cctagaggct cgaccacaga ataatgccag tcaccaccct 2040
   gaacgcacaa tctccagtgc aggatctaat gactgtacat attattgtta ttattattat 2100
   tgttattatt gttgttctgt aaacatgttg cacaagctta gcctttttgc gttctgttgt 2160
   gtgtggctgt aaaaccccat gctttgtgaa atgagaatct tgacattttt cttgtgaaat 2220
   ttggaaaatg tgatcaattg aaatcaactg tgttttgtgt tctctatgtc aaagtttagt 2280
   tttatattga gaatgttaac ttattgcttt gtatcttggg aaaaaaactt tgtaaataag 2340
   ttataaagtt tctttgagac agtaaaatta tgatttcttg aaaaaaaaaa aaaaaaaaaa 2400
   aaa 2403
<210> 10
   <211> 255
   <212> PRT
   <213> Homo sapiens
<220>
   <221> misc_feature
   <223> amino acid sequence of transcript variant one of the DLX1 gene (NM_178120, NP_835221)
<400> 10
   Met Thr Met Thr Thr Met Pro Glu Ser Leu Asn Ser Pro Val Ser Gly
   1 5 10 15
   Lys Ala Val Phe Met Glu Phe Gly Pro Pro Asn Gln Gln Met Ser Pro
               20                  25                  30
   Ser Pro Met Ser His Gly His Tyr Ser Met His Cys Leu His Ser Ala
           35                  40                  45
   Gly His Ser Gln Pro Asp Gly Ala Tyr Ser Ser Ala Ser Ser Phe Ser
       50                  55                  60
   Arg Pro Leu Gly Tyr Pro Tyr Val Asn Ser Val Ser Ser His Ala Ser
   65                  70                  75                  80
   Ser Pro Tyr Ile Ser Ser Val Gln Ser Tyr Pro Gly Ser Ala Ser Leu
                   85                  90                  95
   Ala Gln Ser Arg Leu Glu Asp Pro Gly Ala Asp Ser Glu Lys Ser Thr
               100                 105                 110
   Val Val Glu Gly Gly Glu Val Arg Phe Asn Gly Lys Gly Lys Lys Ile
           115                 120                 125
   Arg Lys Pro Arg Thr Ile Tyr Ser Ser Leu Gln Leu Gln Ala Leu Asn
       130                 135                 140
   Arg Arg Phe Gln Gln Thr Gln Tyr Leu Ala Leu Pro Glu Arg Ala Glu
   145                 150                 155                 160
   Leu Ala Ala Ser Leu Gly Leu Thr Gln Thr Gln Val Lys Ile Trp Phe
                   165                 170                 175
   Gln Asn Lys Arg Ser Lys Phe Lys Lys Leu Met Lys Gln Gly Gly Ala
               180                 185                 190
   Ala Leu Glu Gly Ser Ala Leu Ala Asn Gly Arg Ala Leu Ser Ala Gly
           195                 200                 205
   Ser Pro Pro Val Pro Pro Gly Trp Asn Pro Asn Ser Ser Ser Gly Lys
       210                 215                 220
   Gly Ser Gly Gly Asn Ala Gly Ser Tyr Ile Pro Ser Tyr Thr Ser Trp
   225                 230                 235                 240
   Tyr Pro Ser Ala His Gln Glu Ala Met Gln Gln Pro Gln Leu Met
                   245                 250                 255
<210> 11
   <211> 2203
   <212> DNA
   <213> Homo sapiens
<220>
   <221> misc_feature
   <223> cDNA sequence of transcript variant two of the DLX1 gene (NM_001038493.1, NP_001033582.1)
<400> 11
   aagctttgaa ccgagtttgg ggagctcagc agcatcatgc ttagactttt caaagagaca        60
   aactccattt tcttatgaat ggaaagtgaa aacccctgtt ccgcttaaat tgggttcctt       120
   cctgtcctga gaaacataga gacccccaaa agggaagcag aggagagaaa gtcccacacc       180
   cagaccccgc gagaagagat gaccatgacc accatgccag aaagtctcaa cagccccgtg       240
   tcgggcaagg cggtgtttat ggagtttggg ccgcccaacc agcaaatgtc tccttctccc       300
   atgtcccacg ggcactactc catgcactgt ttacactcgg cgggccattc gcagcccgac       360
   ggcgcctaca gctcagcctc gtccttctcc cgaccgctgg gctaccccta cgtcaactcg       420
   gtcagcagcc acgcatccag cccctacatc agttcggtgc agtcctaccc gggcagcgcc       480
   agcctcgccc agagccgcct ggaggaccca ggtcaagatc tggttccaaa acaagcgatc       540
   caagttcaag aagctgatga agcagggtgg ggcggctctg gagggtagtg cgttggccaa       600
   cggtcgggcc ctgtctgctg gctccccacc cgtgccgccc ggctggaacc ctaactcttc       660
   atccgggaag ggctcaggag gaaacgcggg ctcctatatc cccagctaca catcgtggta      720
   cccttcagcg caccaagaag ctatgcagca accccaactt atgtgaggtt gcccgcccgt      780
   ctccttcttg tctccccggc ccaggtccct cccgcctcca ggtccatcca tcccgtccgg      840
   aaaagaagga cccagaggga agaaggaaca gtggaggcgg gacgccctcc atctcctcgg      900
   agccccgcga ggtccggccc agcaacttcc cggcatccgc gctctagcct gaaccctggc      960
   ctgggccgag cagtggcagc agagagtggc ctcggaggga agccactgcc acctgagaca     1020
   gcccaagcag caagataaac ccgctccacc cgacccgccg accttcagct ttgtgggact     1080
   atcaggaaaa aacaaaacaa aaacaaaatg tagaaaaagc aaaagctctt ttctgtcctg     1140
   tcagtctcct gtctcctttt gctctgtctg tgcgctggta aagtccaggt cctcatccgt     1200
   ccgctgtcct cattctgcgg cctcagcaaa aagccacaag gtctgagcgg cccgggtcct     1260
   gccgggctga ccatctccgg atcctgggac actctgcctg accatctgtg tagctggtgt     1320
   gggaatctgg gggcattgga gggagggggt tttatttatt gagaaatgga cttcgcctga     1380
   ggctgtttgc caattcaggg ttctgctggg cgcaaggaac gcactgttca aacgcactgt     1440
   ttactttaag cgcacgggga gaaacgaata aggaggacgt ggtgattttt aatttataca     1500
   gtaacttttg tacttctctg gtatggagag tttggagccg aatgatttgc attttttaca     1560
   tgtccgacat tatttaataa ataattttta aaagaaaaga acgataaatg aagccaacat     1620
   gattttctca tttcgggagg aactctgttg cttcgcctgg acaagaagga aaatgctgat     1680
   ttcctccttg ggtagaaaga gggagcgagg gcaaatgggg agtagagaga aaacaggcga     1740
   gaacaagcac tctaattcca gtgggcttta aaataagaca aaatcagctt tacaacaatc     1800
   cctagaggct cgaccacaga ataatgccag tcaccaccct gaacgcacaa tctccagtgc     1860
   aggatctaat gactgtacat attattgtta ttattattat tgttattatt gttgttctgt     1920
   aaacatgttg cacaagctta gcctttttgc gttctgttgt gtgtggctgt aaaaccccat     1980
   gctttgtgaa atgagaatct tgacattttt cttgtgaaat ttggaaaatg tgatcaattg     2040
   aaatcaactg tgttttgtgt tctctatgtc aaagtttagt tttatattga gaatgttaac     2100
   ttattgcttt gtatcttggg aaaaaaactt tgtaaataag ttataaagtt tctttgagac     2160
   agtaaaatta tgatttcttg aaaaaaaaaa aaaaaaaaaa aaa                      2203
<210> 12
   <211> 129
   <212> PRT
   <213> Homo sapiens
<220>
   <221> misc_feature
   <223> amino acid sequence of transcript variant two of the DLX1 gene (NM_001038493.1, NP_001033582.1)
<400> 12
   Met Thr Met Thr Thr Met Pro Glu Ser Leu Asn Ser Pro Val Ser Gly
   1               5                   10                  15
   Lys Ala Val Phe Met Glu Phe Gly Pro Pro Asn Gln Gln Met Ser Pro
               20                  25                  30
   Ser Pro Met Ser His Gly His Tyr Ser Met His Cys Leu His Ser Ala
           35                  40                  45
   Gly His Ser Gln Pro Asp Gly Ala Tyr Ser Ser Ala Ser Ser Phe Ser
       50                  55                  60
   Arg Pro Leu Gly Tyr Pro Tyr Val Asn Ser Val Ser Ser His Ala Ser
   65                  70                  75                  80
   Ser Pro Tyr Ile Ser Ser Val Gln Ser Tyr Pro Gly Ser Ala Ser Leu
                   85                  90                  95
   Ala Gln Ser Arg Leu Glu Asp Pro Gly Gln Asp Leu Val Pro Lys Gln
               100                 105                 110
   Ala Ile Gln Val Gln Glu Ala Asp Glu Ala Gly Trp Gly Gly Ser Gly
   115 120 125
   Gly
<210> 13
   <211> 4510
   <212> DNA
   <213> Homo sapiens
<220>
   <221> misc_feature
   <223> cDNA sequence of the TDRD1 gene (NM_198795.1, NP_942090.1)
<400> 13
   gctgaggcca ggagggcgca ctggggattg gaggcgaggg aagtgcaggg cgcatcccag      60
   gcggcagggc tcccagcatc ggcagtcgcc atcaccgcca gaccgcagag acaggttcgg     120
   atccgcggtc ctcttgcctc tttccaggcc tcgatgagtg ttaaatcgcc atttaatgtg     180
   atgtcaagaa ataatttgga agcacctcct tgtaagatga cagagccatt taattttgag     240
   aaaaatgaaa acaagcttcc accacatgag tctttaagaa gtcctggaac acttcctaac     300
   caccctaatt tcaggctgaa aagctcagag aatggaaata aaaagaacaa ttttttgctt    360
   tgtgagcaaa ccaaacaata tttggctagt caggaagaca attcagtttc ttcaaacccg     420
   aatggcatca acggagaagt agttggctcc aaaggagaca ggaaaaaatt gccagcagga     480
   aactcagtgt caccaccaag tgctgaaagt aattcaccac ccaaagaagt gaatattaag     540
   cctggaaata atgtacgtcc tgcaaaatca aaaaaactaa acaagttggt cgagaattcc     600
   ttgtccataa gtaatccagg gctcttcacc tccttaggac ctcctcttcg gtccacaact     660
   tgccatcgct gtggcctatt tggatcgctg aggtgctctc agtgcaagca gacctactat     720
   tgctccacag catgtcaaag aagagactgg tctgcacaca gcatcgtgtg caggcctgtt     780
   cagccaaatt tccacaaact tgaaaataaa tcatctattg aaacaaagga tgtggaggta     840
   aacaataaga gtgactgtcc acttggagtt actaaggaaa tagccatttg ggctgagaga     900
   ataatgtttt ctgatttgag aagtctacaa ctcaagaaaa ccatggaaat aaagggtacg     960
   gttaccgaat tcaaacaccc aggggacttc tacgtgcagt tatattcttc agaagtttta    1020
   gaatacatga accaactctc tgccagctta aaagaaacat atgcaaatgt gcatgaaaaa    1080
   gactatattc ctgttaaggg ggaagtttgt attgccaagt acactgttga tcagacctgg    1140
   aacagagcaa tcatacaaaa cgttgatgtg cagcaaaaga aggcacatgt cttatatatt    1200
   gattatggaa atgaagaaat aattccatta aacagaattt accacctcaa caggaacatt    1260
   gacttgtttc ctccttgtgc cataaagtgc tttgtagcca atgttatccc agcagaaggg    1320
   aattggagca gtgattgtat caaagctact aaaccactgt taatggagca gtactgctcc    1380
   ataaagattg tcgacatctt ggaagaggaa gtggttacct ttgctgtaga agttgagctg    1440
   ccaaattcag gaaaactttt agaccatgtg cttatagaaa tgggatatgg cttgaaaccc    1500
   agtggacaag attctaagaa ggaaaatgca gatcaaagtg atcctgaaga tgttggaaaa    1560
   atgacaactg aaaacaacat tgtcgtagac aaaagtgacc taatcccaaa agtgttaact    1620
   ttgaatgtag gtgatgagtt ttgtggtgtg gttgcccaca ttcaaacacc agaagacttc    1680
   ttttgtcaac aactgcaaag tggccgaaag cttgctgaac ttcaggcatc ccttagcaag    1740
   tactgtgatc agttgcctcc acgctctgat ttttatccag ccattggtga tatatgttgt   1800
   gctcagttct cagaggatga tcagtggtac cgtgcctctg ttttggctta cgcttctgaa    1860
   gaatctgtac tggtcggata tgtagattat ggaaactttg aaatccttag tttgatgaga    1920
   ctttgtccca taatcccaaa gttgttggaa ttgccaatgc aagctataaa gtgtgtacta    1980
   gcaggagtaa agccatcatt aggaatttgg actccagaag ctatttgtct catgaaaaaa    2040
   cttgtacaga acaaaataat cacagtgaaa gtggtggaca agttggaaaa cagttccctg    2100
   gtggagctta ttgataaatc cgagacgcct catgtcagtg ttagcaaagt tctcctagat    2160
   gcaggctttg ctgtgggaga acagagtatg gtgacagata aacccagtga cgtgaaagaa    2220
   accagtgttc ccttgggtgt ggaaggaaaa gtaaatccat tggagtggac atgggttgaa    2280
   cttggtgttg accaaacagt agatgttgtg gtctgtgtga tatatagtcc tggagaattt    2340
   tattgccatg tgcttaaaga ggatgcttta aagaaactca atgatttgaa caagtcatta     2400
   gcagaacact gccagcagaa gttacctaat ggtttcaagg cagagatagg acaaccttgt     2460
   tgtgcttttt ttgcaggtga tggtagttgg tatcgtgctt tagtcaagga aatcttacca     2520
   aatggacatg ttaaagtaca ttttgtggat tatggaaaca tcgaagaagt tactgcagat     2580
   gaactccgaa tgatatcatc aacattttta aaccttccct ttcagggaat acggtgccag     2640
   ttagcagata tacagtctag aaacaaacat tggtctgaag aagccataac aagattccag     2700
   atgtgtgttg ctgggataaa attgcaagcc agagtggttg aagtcactga aaatgggata     2760
   ggagttgaac tcaccgatct ctccacttgt tatcccagaa taattagtga tgttctgatt     2820
   gatgaacatc tggttttaaa atctgcttca ccacataaag acttaccaaa tgacagactt     2880
   gttaataaac atgagcttca agttcatgta cagggacttc aagctacctc ttcagctgag     2940
   caatggaaga cgatagaatt gccagtggat aaaactatac aagcaaatgt attagaaatc     3000
   ataagcccaa acttgtttta tgctctacca aaagggatgc cagaaaatca ggaaaagctg     3060
   tgcatgttga cagctgaatt attagaatac tgcaatgctc cgaaaagtcg accaccctat     3120
   agaccaagaa ttggagacgc atgctgtgcc aaatacacaa gtgatgattt ttggtatcgt     3180
   gcagttgttc tggggacatc agacactgat gtggaagtgc tctatgcaga ctatggaaac     3240
   attgaaaccc tgcctctttg cagagtgcaa ccaatcacct ctagccacct ggcgcttcct     3300
   ttccaaatta ttagatgttc acttgaagga ttaatggaat tgaatggaag ctcttctcaa     3360
   ttaataataa tgctattaaa aaatttcatg ttgaatcaga atgtaatgct ttctgtgaaa     3420
   ggaattacaa agaatgtcca tacagtgtca gttgagaaat gttctgagaa tgggactgtc     3480
   gatgtagctg ataagctagt gacatttggt ctggcaaaaa acatcacacc tcaaaggcag     3540
   agtgctttaa atacagaaaa gatgtatagg atgaattgct gctgcacaga gttacagaaa     3600
   caagttgaaa aacatgaaca tattcttctc ttcctcttaa acaattcaac caatcaaaat     3660
   aaatttattg aaatgaaaaa actgttaaaa aaaacagcat ctcttggagg taaaccctta     3720
   tgagacagga aacagcaaag gctagcttta ggagagaaag tacagcacct ggtgttttta     3780
   tttatgagaa ccttttcttt gtccactttc tctgtaatga ccttctatcc ctccgttttt     3840
   gcctgcctgc cattctccta ttaggttggt ggtttttatt ttcctctaag ttccttccac     3900
   caaataaata ttacgtaaaa aattcatacc aaatcaatga gaatactggc aaggaataca     3960
   tagggacttt ctgctatata tgtaactttt tattacttaa aggtaccgaa ggaaggccag     4020
   gtgcagtggc tcacgcccag cactttggga ggctgaggtg ggaggatccc ttgaggccag     4080
   gagttcaagg ttacagtgag ctatgatagt gccactgcac tccagcctgg gtgacagatt     4140
   ttgtcttaaa aaaaaaaaaa aaaaagttga tatgagtttt attttctgtc cgtttgaaat     4200
   attttgtaat attccctgca ttctctgtcg tctgcctctt ccacataatg tcctttgctt     4260
   tcatgtttgt tatcttcttt ttctgttcac tcagaggtca tcaatttctt tctctccgtc     4320
   cttaattgga ttatttttct tttggccttt gggcacagag tctgacctct ggaccactct     4380
   aactggagaa ggaactttat gttccctctc ctgctgtgtc cacaacctta gaaatctgta     4440
   gctagatttt tgttgttata gatagaattt actgtttctg aaacccaaat acagttatca     4500
   gtttaaggtt                                                            4510
<210> 14
   <211> 1189
   <212> PRT
   <213> Homo sapiens
<220>
   <221> misc_feature
   <223> amino acid sequence of the TDRD1 gene (NM_198795.1, NP_942090.1)
<400> 14
   Met Ser Val Lys Ser Pro Phe Asn Val Met Ser Arg Asn Asn Leu Glu
   1               5                   10                  15
   Ala Pro Pro Cys Lys Met Thr Glu Pro Phe Asn Phe Glu Lys Asn Glu
               20                  25                  30
   Asn Lys Leu Pro Pro His Glu Ser Leu Arg Ser Pro Gly Thr Leu Pro
           35                  40                  45
   Asn His Pro Asn Phe Arg Leu Lys Ser Ser Glu Asn Gly Asn Lys Lys
       50                  55                  60
   Asn Asn Phe Leu Leu Cys Glu Gln Thr Lys Gln Tyr Leu Ala Ser Gln
   65                  70                  75                  80
   Glu Asp Asn Ser Val Ser Ser Asn Pro Asn Gly Ile Asn Gly Glu Val
                   85                  90                  95
   Val Gly Ser Lys Gly Asp Arg Lys Lys Leu Pro Ala Gly Asn Ser Val
               100                 105                 110
   Ser Pro Pro Ser Ala Glu Ser Asn Ser Pro Pro Lys Glu Val Asn Ile
           115                 120                 125
   Lys Pro Gly Asn Asn Val Arg Pro Ala Lys Ser Lys Lys Leu Asn Lys
       130                 135                 140
   Leu Val Glu Asn Ser Leu Ser Ile Ser Asn Pro Gly Leu Phe Thr Ser
   145                 150                 155                 160
   Leu Gly Pro Pro Leu Arg Ser Thr Thr Cys His Arg Cys Gly Leu Phe
                   165                 170                 175
   Gly Ser Leu Arg Cys Ser Gln Cys Lys Gln Thr Tyr Tyr Cys Ser Thr
               180                 185                 190
   Ala Cys Gln Arg Arg Asp Trp Ser Ala His Ser Ile Val Cys Arg Pro
           195                 200                 205
   Val Gln Pro Asn Phe His Lys Leu Glu Asn Lys Ser Ser Ile Glu Thr
       210                 215                 220
   Lys Asp Val Glu Val Asn Asn Lys Ser Asp Cys Pro Leu Gly Val Thr
   225                 230                 235                 240
   Lys Glu Ile Ala Ile Trp Ala Glu Arg Ile Met Phe Ser Asp Leu Arg
                   245                 250                 255
   Ser Leu Gln Leu Lys Lys Thr Met Glu Ile Lys Gly Thr Val Thr Glu
               260                 265                 270
   Phe Lys His Pro Gly Asp Phe Tyr Val Gln Leu Tyr Ser Ser Glu Val
           275                 280                 285
   Leu Glu Tyr Met Asn Gln Leu Ser Ala Ser Leu Lys Glu Thr Tyr Ala
       290                 295                 300
   Asn Val His Glu Lys Asp Tyr Ile Pro Val Lys Gly Glu Val Cys Ile
   305                 310                 315                 320
   Ala Lys Tyr Thr Val Asp Gln Thr Trp Asn Arg Ala Ile Ile Gln Asn
                   325                 330                 335
   Val Asp Val Gln Gln Lys Lys Ala His Val Leu Tyr Ile Asp Tyr Gly
               340                 345                 350
   Asn Glu Glu Ile Ile Pro Leu Asn Arg Ile Tyr His Leu Asn Arg Asn
           355                 360                 365
   Ile Asp Leu Phe Pro Pro Cys Ala Ile Lys Cys Phe Val Ala Asn Val
       370                 375                 380
   Ile Pro Ala Glu Gly Asn Trp Ser Ser Asp Cys Ile Lys Ala Thr Lys
   385                 390                 395                 400
   Pro Leu Leu Met Glu Gln Tyr Cys Ser Ile Lys Ile Val Asp Ile Leu
                   405                 410                 415
   Glu Glu Glu Val Val Thr Phe Ala Val Glu Val Glu Leu Pro Asn Ser
               420                 425                 430
   Gly Lys Leu Leu Asp His Val Leu Ile Glu Met Gly Tyr Gly Leu Lys
           435                 440                 445
   Pro Ser Gly Gln Asp Ser Lys Lys Glu Asn Ala Asp Gln Ser Asp Pro
       450                 455                 460
   Glu Asp Val Gly Lys Met Thr Thr Glu Asn Asn Ile Val Val Asp Lys
   465                 470                 475                 480
   Ser Asp Leu Ile Pro Lys Val Leu Thr Leu Asn Val Gly Asp Glu Phe
                   485                 490                 495
   Cys Gly Val Val Ala His Ile Gln Thr Pro Glu Asp Phe Phe Cys Gln
               500                 505                 510
   Gln Leu Gln Ser Gly Arg Lys Leu Ala Glu Leu Gln Ala Ser Leu Ser
           515                 520                 525
   Lys Tyr Cys Asp Gln Leu Pro Pro Arg Ser Asp Phe Tyr Pro Ala Ile
       530                 535                 540
   Gly Asp Ile Cys Cys Ala Gln Phe Ser Glu Asp Asp Gln Trp Tyr Arg
   545                 550                 555                 560
   Ala Ser Val Leu Ala Tyr Ala Ser Glu Glu Ser Val Leu Val Gly Tyr
                   565                 570                 575
   Val Asp Tyr Gly Asn Phe Glu Ile Leu Ser Leu Met Arg Leu Cys Pro
               580                 585                 590
   Ile Ile Pro Lys Leu Leu Glu Leu Pro Met Gln Ala Ile Lys Cys Val
           595                 600                 605
   Leu Ala Gly Val Lys Pro Ser Leu Gly Ile Trp Thr Pro Glu Ala Ile
       610                 615                 620
   Cys Leu Met Lys Lys Leu Val Gln Asn Lys Ile Ile Thr Val Lys Val
   625                 630                 635                 640
   Val Asp Lys Leu Glu Asn Ser Ser Leu Val Glu Leu Ile Asp Lys Ser
                   645                 650                 655
   Glu Thr Pro His Val Ser Val Ser Lys Val Leu Leu Asp Ala Gly Phe
               660                 665                 670
   Ala Val Gly Glu Gln Ser Met Val Thr Asp Lys Pro Ser Asp Val Lys
           675                 680                 685
   Glu Thr Ser Val Pro Leu Gly Val Glu Gly Lys Val Asn Pro Leu Glu
       690                 695                 700
   Trp Thr Trp Val Glu Leu Gly Val Asp Gln Thr Val Asp Val Val Val
   705                 710                 715                 720
   Cys Val Ile Tyr Ser Pro Gly Glu Phe Tyr Cys His Val Leu Lys Glu
                   725                 730                 735
   Asp Ala Leu Lys Lys Leu Asn Asp Leu Asn Lys Ser Leu Ala Glu His
               740                 745                 750
   Cys Gln Gln Lys Leu Pro Asn Gly Phe Lys Ala Glu Ile Gly Gln Pro
           755                 760                 765
   Cys Cys Ala Phe Phe Ala Gly Asp Gly Ser Trp Tyr Arg Ala Leu Val
       770                 775                 780
   Lys Glu Ile Leu Pro Asn Gly His Val Lys Val His Phe Val Asp Tyr
   785                 790                 795                 800
   Gly Asn Ile Glu Glu Val Thr Ala Asp Glu Leu Arg Met Ile Ser Ser
                   805                 810                 815
   Thr Phe Leu Asn Leu Pro Phe Gln Gly Ile Arg Cys Gln Leu Ala Asp
               820                 825                 830
   Ile Gln Ser Arg Asn Lys His Trp Ser Glu Glu Ala Ile Thr Arg Phe
           835                 840                 845
   Gln Met Cys Val Ala Gly Ile Lys Leu Gln Ala Arg Val Val Glu Val
       850                 855                 860
   Thr Glu Asn Gly Ile Gly Val Glu Leu Thr Asp Leu Ser Thr Cys Tyr
   865                 870                 875                 880
   Pro Arg Ile Ile Ser Asp Val Leu Ile Asp Glu His Leu Val Leu Lys
                   885                 890                 895
   Ser Ala Ser Pro His Lys Asp Leu Pro Asn Asp Arg Leu Val Asn Lys
               900                 905                 910
   His Glu Leu Gln Val His Val Gln Gly Leu Gln Ala Thr Ser Ser Ala
           915                 920                 925
   Glu Gln Trp Lys Thr Ile Glu Leu Pro Val Asp Lys Thr Ile Gln Ala
       930                 935                 940
   Asn Val Leu Glu Ile Ile Ser Pro Asn Leu Phe Tyr Ala Leu Pro Lys
   945                 950                 955                 960
   Gly Met Pro Glu Asn Gln Glu Lys Leu Cys Met Leu Thr Ala Glu Leu
                   965                 970                 975
   Leu Glu Tyr Cys Asn Ala Pro Lys Ser Arg Pro Pro Tyr Arg Pro Arg
               980                 985                 990
   Ile Gly Asp Ala Cys Cys Ala Lys  Tyr Thr Ser Asp Asp  Phe Trp Tyr
           995                 1000                1005
   Arg Ala  Val Val Leu Gly Thr  Ser Asp Thr Asp Val  Glu Val Leu
       1010                1015                1020
   Tyr Ala  Asp Tyr Gly Asn Ile  Glu Thr Leu Pro Leu  Cys Arg Val
       1025                1030                1035
   Gln Pro  Ile Thr Ser Ser His  Leu Ala Leu Pro Phe  Gln Ile Ile
       1040                1045                1050
   Arg Cys  Ser Leu Glu Gly Leu  Met Glu Leu Asn Gly  Ser Ser Ser
       1055                1060                1065
   Gln Leu  Ile Ile Met Leu Leu  Lys Asn Phe Met Leu  Asn Gln Asn
       1070                1075                1080
   Val Met  Leu Ser Val Lys Gly  Ile Thr Lys Asn Val  His Thr Val
       1085                1090                1095
   Ser Val  Glu Lys Cys Ser Glu  Asn Gly Thr Val Asp  Val Ala Asp
       1100                1105                1110
   Lys Leu  Val Thr Phe Gly Leu  Ala Lys Asn Ile Thr  Pro Gln Arg
       1115                1120                1125
   Gln Ser  Ala Leu Asn Thr Glu  Lys Met Tyr Arg Met  Asn Cys Cys
       1130                1135                1140
   Cys Thr  Glu Leu Gln Lys Gln  Val Glu Lys His Glu  His Ile Leu
       1145                1150                1155
   Leu Phe  Leu Leu Asn Asn Ser  Thr Asn Gln Asn Lys  Phe Ile Glu
       1160                1165                1170
   Met Lys  Lys Leu Leu Lys Lys  Thr Ala Ser Leu Gly  Gly Lys Pro
       1175                1180                1185
   Leu